(19) Europäisches Patentamt
European Patent Office
Office européen des brevets

(11) Veröffentlichungsnummer: **0 607 825 A1**

# (12) EUROPÄISCHE PATENTANMELDUNG

(21) Anmeldenummer: **94100204.0**

(22) Anmeldetag: **07.01.94**

(51) Int. Cl.5: **C07D 471/04**, A61K 31/47, C07D 401/04, C07D 487/04, C07D 498/04

(30) Priorität: **19.01.93 DE 4301246**

(43) Veröffentlichungstag der Anmeldung: **27.07.94 Patentblatt 94/30**

(84) Benannte Vertragsstaaten: **AT BE CH DE DK ES FR GB GR IE IT LI LU MC NL PT SE**

(71) Anmelder: **BAYER AG**

**D-51368 Leverkusen(DE)**

(72) Erfinder: **Bartel, Stephan, Dr.**
**Margarethenhöhe 7**
**D-51465 Bergisch Gladbach(DE)**
Erfinder: **Krebs, Andreas, Dr.**
**Am Gartenfeld 70**
**D-51519 Odenthal(DE)**
Erfinder: **Kunisch, Franz, Dr.**
**Zum Hahnenberg 20**
**D-51519 Odenthal(DE)**
Erfinder: **Petersen, Uwe, Dr.**
**Auf dem Forst 4**
**D-51375 Leverkusen(DE)**
Erfinder: **Schenke, Thomas, Dr.**
**Mühlenstrasse 113**
**D-51469 Bergisch Gladbach(DE)**
Erfinder: **Grohe, Klaus, Dr.**
**Am Wasserturm 10**
**D-51519 Odenthal(DE)**
Erfinder: **Schriewer, Michael, Dr.**
**Am Thelen Siefen 1a**
**D-51519 Odenthal(DE)**
Erfinder: **Bremm, Klaus-Dieter, Dr.**
**Girardetstrasse 120**
**D-42109 Wuppertal(DE)**
Erfinder: **Endermann, Rainer, Dr.**
**In den Birken 152a**
**D-42113 Wuppertal(DE)**
Erfinder: **Metzger, Karl Georg, Dr.**
**Pahlkestrasse 75**
**D-42115 Wuppertal(DE)**

## (54) 6-H-4-Oxo-3-chinolinecarbonsäure und ihre Analoge als antibakterielle Mittel.

(57) Die Erfindung betrifft neue Chinolon- und Naphthyridoncarbonsäurederivate, die in der 6-Position Wasserstoff tragen der allgemeinen Formel I

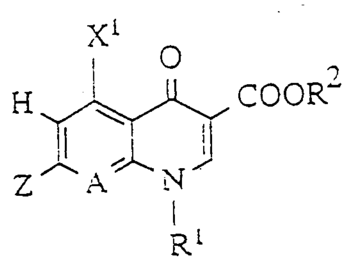

(I),

in welcher

Z für Reste der Strukturen

EP 0 607 825 A1

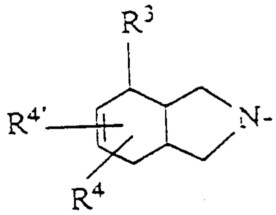

,

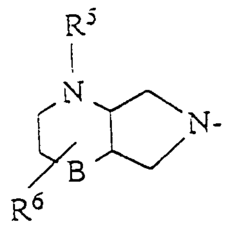

steht,
der 6-Position Wasserstoff tragen, Verfahren zu ihrer Herstellung sowie diese enthaltende antibakterielle Mittel und Futterzusatzstoffe.

Die Erfindung betrifft neue Chinolon- und Naphthyridoncarbonsäurederivate, die in der 6-Position Wasserstoff tragen, Verfahren zu ihrer Herstellung sowie diese enthaltende antibakterielle Mittel und Futterzusatzstoffe.

Es ist bereits bekannt geworden, daß derartige Chinoloncarbonsäuren antibakteriell wirksam sind. Beispiele hierfür finden sich in US 4.416.884, EP-A 393 400, 8-Methyl-7-piperazinyl-chinoloncarbonsäuren wurden in DE-A 3 007 006 und 7-(3-Aminopyrrolidinyl)-8-fluorchinoloncarbonsäuren im Journal of Medicinal Chemistry 35, 198 (1992) beschrieben.

Es wurden nun Verbindungen der allgemeinen Formel (I) gefunden

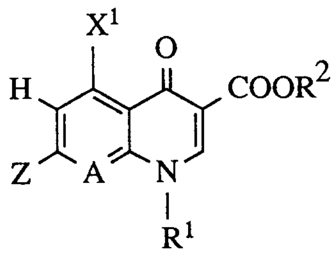

(I),

in welcher

R¹ für gegebenenfalls durch Hydroxy, Halogen oder $C_1$-$C_3$-Alkoxy substituiertes geradkettiges oder verzweigtes $C_1$-$C_4$-Alkyl, für gegebenenfalls durch $C_1$-$C_3$-Alkyl oder Halogen substituiertes $C_3$-$C_6$-Cycloalkyl, $C_2$-$C_4$-Alkenyl, ferner für $C_1$-$C_3$-Alkoxy, Amino, Monoalkylamino mit 1 bis 3 C-Atomen, Dialkylamino mit 1 bis 3 C-Atomen je Alkylgruppe oder für gegebenenfalls durch Halogen ein- bis dreifach substituiertes Phenyl steht,

$R^2$ für Wasserstoff, gegebenenfalls durch Hydroxy, Methoxy, Amino, Methylamino oder Dimethylamino substituiertes Alkyl mit 1 bis 4 Kohlenstoffatomen oder (5-Methyl-2-oxo-1,3-dioxol-4-yl)-methyl,

$X^1$ für Wasserstoff, Halogen, Amino, Methyl oder Trifluormethyl,

Z für Reste der Strukturen

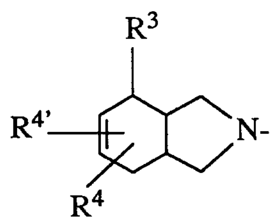

,

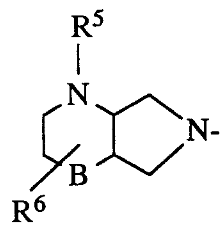

steht,

worin

$R^3$ für Wasserstoff, Hydroxy, $-NR^7R^8$, Hydroxymethyl oder $-CH_2-NR^7R^8$ steht,

wobei

$R^7$ Wasserstoff, gegebenenfalls durch Hydroxy substituiertes $C_1$-$C_3$-Alkyl, Alkoxycarbonyl mit 1 bis 4 C-Atomen im Alkoxyteil oder $C_1$-$C_3$-Acyl und

$R^8$ Wasserstoff oder Methyl bedeutet,

$R^4$ für Wasserstoff, geradkettiges oder verzweigtes $C_1$-$C_3$-Alkyl oder Cyclopropyl,

$R^{4'}$ für Wasserstoff oder Methyl,

$R^5$ für Wasserstoff oder Methyl,

$R^6$ für Wasserstoff, Methyl oder Reste der Strukturen $-CH=CH-CO_2R^{5'}$, $-CH_2-CH_2-CO_2R^{5'}$, $-CH_2-CO-CH_3$, $-CH_2-CH_2-CN$,

$R^{5'}$ für Methyl oder Ethyl,

B für $-CH_2-$, O oder eine direkte Bindung steht und

A für N oder C-$R^9$ steht, worin

$R^9$ für H, Halogen, Methyl, Trifluormethyl, Vinyl, Ethinyl, Hydroxy oder Methoxy steht oder auch gemeinsam mit $R^1$ eine Brücke der Struktur

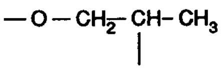 , 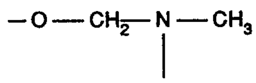 , 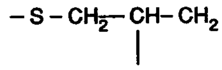 oder 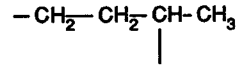

bilden kann,

in Form von Racematen oder als enantiomerenreine Verbindungen, deren pharmazeutisch verwendbaren Hydrate und Säureadditionsalze sowie die Alkali-, Erdalkali-, Silber- und Guanidiniumsalze der zugrundeliegenden Carbonsäuren. Die erfindungsgemäßen Verbindungen weisen im Vergleich zu bekannten Vertretern dieses Strukturtyps eine höhere antibakterielle Wirkung insbesondere im grampositiven Bereich auf.

Sie eignen sich daher als Wirkstoffe für die Human- und Veterinärmedizin, wobei zur Veterinärmedizin auch die Behandlung von Fischen zur Therapie oder zur Vorbeugung bakterieller Infektionen zu zählen ist.

Bevorzugt sind Verbindungen der Formel (I), in welcher

$R^1$ für gegebenenfalls durch Hydroxy oder Fluor substituiertes $C_1$-$C_2$-Alkyl, für gegebenenfalls durch Fluor substituiertes $C_3$-$C_5$-Cycloalkyl, Vinyl, Amino, Monoalkylamino mit 1 bis 2 C-Atomen, Dialkylamino mit 1 bis 2 C-Atomen je Alkylgruppe oder für gegebenenfalls durch Halogen ein- bis zweifach substituiertes Phenyl steht,

$R^2$ für Wasserstoff, gegebenenfalls durch Amino oder Dimethylamino substituiertes Alkyl mit 1 bis 2 Kohlenstoffatomen oder (5-Methyl-2-oxo-1,3-dioxol-4-yl)-methyl,

$X^1$ für Wasserstoff, Fluor, Chlor, Trifluormethyl oder Amino,

Z für Reste der Strukturen

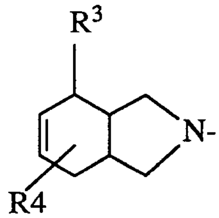 , 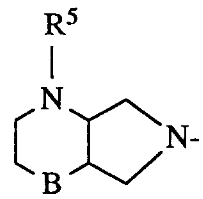

steht,

worin

$R^3$ für Wasserstoff, Hydroxy, $-NR^7R^8$, Hydroxymethyl oder $-CH_2-NR^7R^8$ steht,

wobei

$R^7$ Wasserstoff, gegebenenfalls durch Hydroxy substituiertes $C_1$-$C_2$-Alkyl, Alkoxycarbonyl mit 1 bis 4 C-Atomen im Alkoxyteil oder $C_1$-$C_3$-Acyl und

$R^8$ Wasserstoff oder Methyl bedeutet,

$R^4$ für Wasserstoff, geradkettiges oder verzweigtes $C_1$-$C_3$-Alkyl oder Cyclopropyl,

$R^5$ für Wasserstoff oder Methyl,

B für $-CH_2-$, O oder eine direkte Bindung steht und

A für N oder $C-R^9$ steht, worin

$R^9$ für H, Chlor, Fluor, Methyl, Trifluormethyl, Hydroxy oder Methoxy steht oder auch gemeinsam mit $R^1$ eine Brücke der Struktur

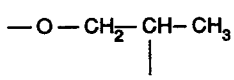 oder 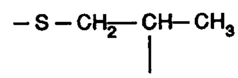

bilden kann,

und deren pharmazeutisch verwendbaren Hydrate und Säureadditionssalze sowie die Alkali-, Erdalkali-, Silber- und Guanidiniumsalze der zugrundeliegenden Carbonsäuren.

Besonders bevorzugt sind die Verbindungen der Formel (I), in welcher

$R^1$ für Methyl, Ethyl, gegebenenfalls durch Fluor substituiertes Cyclopropyl oder für gegebenenfalls durch Fluor ein- oder zweifach substituiertes Phenyl steht,

$R^2$ für Wasserstoff, Methyl oder Ethyl,

$X^1$ für Wasserstoff, Fluor, Trifluormethyl oder Amino,

Z für Reste der Strukturen

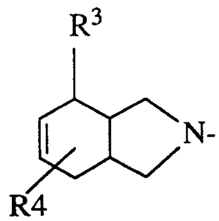 , 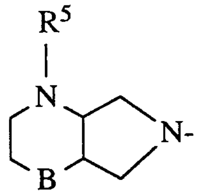

steht,
worin

$R^3$ für Wasserstoff, Hydroxy, $-NR^7R^8$, Hydroxymethyl oder $-CH_2-NR^7R^8$ steht,
wobei

$R^7$ Wasserstoff, Methyl, Alkoxycarbonyl mit 1 bis 4 C-Atomen im Alkoxyteil oder $C_1$-$C_3$-Acyl und

$R^8$ Wasserstoff oder Methyl bedeutet,

$R^4$ für Wasserstoff, geradkettiges oder verzweigtes $C_1$-$C_3$-Alkyl oder Cyclopropyl,

$R^5$ für Wasserstoff oder Methyl,

B für $-CH_2-$, O oder eine direkte Bindung steht und

A für N oder C-$R^9$ steht, worin

$R^9$ für H, Chlor, Fluor, Methyl, Methoxy, Trifluormethyl steht oder auch gemeinsam mit $R^1$ eine Brücke der Struktur

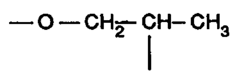

bilden kann,

und deren pharmazeutisch verwendbaren Hydrate und Säureadditionssalze sowie die Alkali-, Erdalkali-, Silber- und Guanidiniumsalze der zugrundeliegenden Carbonsäuren.

Weiterhin wurde gefunden, daß man Verbindungen der Formel (I) erhält, wenn man Verbindungen der Formel (II)

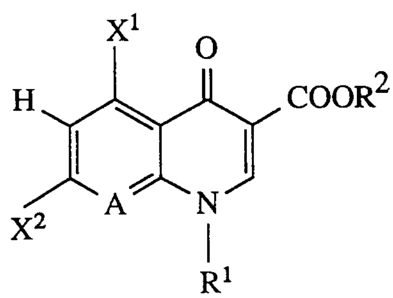 (II),

in welcher

$R^1$, $R^2$, $X^1$ und A die oben angegebene Bedeutung haben und

$X^2$ für Halogen, insbesondere Fluor oder Chlor, steht,

mit Verbindungen der Formel (III)

Z-H (III),

in welcher

Z die oben angegebene Bedeutung hat,

gegebenenfalls in Gegenwart von Säurefängern umsetzt.

Verwendet man beispielsweise 1-Cyclopropyl-7,8-difluor-1,4-dihydro-4-oxo-3-chinolincarbonsäure und 4-Methylamino-1,3,3a,4,7,7a-hexahydroisoindol, so kann der Reaktionsverlauf durch das folgende Formelschema wiedergegeben werden:

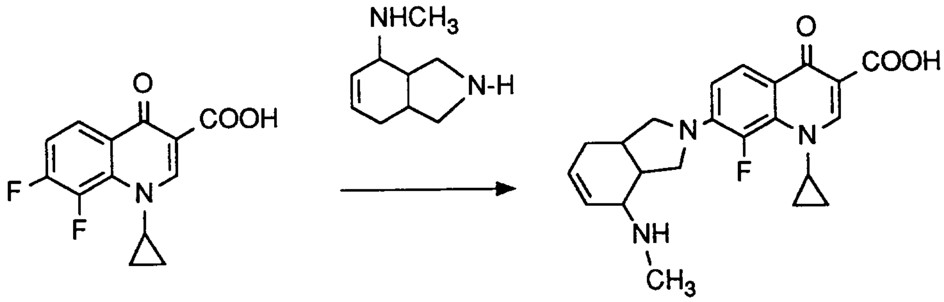

Die als Ausgangsverbindungen verwendeten Verbindungen der Formel (II) sind bekannt, bzw. können nach bekannten Verfahren hergestellt werden. Sie können gegebenenfalls als Racemate, Enantiomere oder reine Diastereomere eingesetzt werden.

Als Beispiele seien genannt:
1-Cyclopropyl-7,8-difluor-1,4-dihydro-4-oxo-3-chinolincarbonsäure,
7-Chlor-1-cyclopropyl-1,4-dihydro-4-oxo-3-chinolincarbonsäure,
7-Chlor-1-ethyl-8-fluor-1,4-dihydro-4-oxo-3-chinolincarbonsäure,
1-Ethyl-7-fluor-1,4-dihydro-4-oxo-3-chinolincarbonsäure,
7-Chlor-1-ethyl-1,4-dihydro-4-oxo-3-chinolincarbonsäure,
7,8-Dichlor-1-ethyl-1,4-dihydro-4-oxo-3-chinolincarbonsäure,
1-Ethyl-7-fluor-8-methyl-1,4-dihydro-4-oxo-3-chinolincarbonsäure,
7-Chlor-1-(4-fluorphenyl)-1,4-dihydro-5-methyl-4-oxo-1,8-naphthyridin-3-carbonsäureethylester,
8-Chlor-1-cyclopropyl-5,7-difluor-1,4-dihydro-4-oxo-3-chinolincarbonsäureethylester,
5-Amino-1-cyclopropyl-7,8-difluor-1,4-dihydro-4-oxo-3-chinolincarbonsäureethylester,
1-Cyclopropyl-5,7,8-trifluor-1,4-dihydro-4-oxo-3-chinolincarbonsäureethylester,
1-Cyclopropyl-7-fluor-1,4-dihydro-8-methyl-4-oxo-3-chinolincarbonsäure,
1-Cyclopropyl-7,8-difluor-1,4-dihydro-5-methyl-4-oxo-3-chinolincarbonsäure,
5-Brom-1-cyclopropyl-7,8-difluor-1,4-dihydro-4-oxo-3-chinolincarbonsäure,
5-Chlor-1-cyclopropyl-7,8-difluor-1,4-dihydro-4-oxo-3-chinolincarbonsäure,
1-Cyclopropyl-7-fluor-1,4-dihydro-8-methoxy-4-oxo-3-chinolincarbonsäure,
7,8-Difluor-1-(2,4-difluorphenyl)-1,4-dihydro-4-oxo-3-chinolincarbonsäure,
8-Chlor-7-fluor-1-(2,4-difluorphenyl)-1,4-dihydro-4-oxo-3-chinolincarbonsäure,
7-Fluor-1-(2,4-difluorphenyl)-1,4-dihydro-4-oxo-3-chinolincarbonsäure,
10-Fluor-2,3-dihydro-3-methyl-7-oxo-7H-pyrido[1,2,3-d,e][1,4]benzoxazin-6-carbonsäure,
10-Chlor-2,3-dihydro-3-methyl-7-oxo-7H-pyrido[1,2,3-d,e][1,4]benzoxazin-6-carbonsäure,
10-Chlor-8-fluor-2,3-dihydro-3-methyl-7-oxo-7H-pyrido[1,2,3-d,e][1,4]benzoxazin-6-carbonsäure,
10-Chlor-2,3-dihydro-3-methyl-8-nitro-7-oxo-7H-pyrido[1,2,3-d,e][1,4]benzoxazin-6-carbonsäure,
10-Chlor-2,3-dihydro-3,8-dimethyl-7-oxo-7H-pyrido[1,2,3-d,e][1,4]benzoxazin-6-carbonsäure,
8-Chlor-6,7-dihydro-1-oxo-1H, 5H-benzo[ij]chinolizin-2-carbonsäure,
8-Chlor-1-cyclopropyl-7-fluor-1,4-dihydro-4-oxo-3-chinolincarbonsäure,
7,8-Difluor-1-(2-fluorcyclopropyl)-1,4-dihydro-4-oxo-3-chinolincarbonsäure,
8-Chlor-7-fluor-1-(2-fluorcyclopropyl)-1,4-dihydro-4-oxo-3-chinolincarbonsäure,
1-Cyclopropyl-7,8-difluor-5-trifluormethyl-1,4-dihydro-4-oxo-3-chinolincarbonsäure,
1-Cyclopropyl-5,7-difluor-8-trifluormethyl-1,4-dihydro-4-oxo-3-chinolincarbonsäure,
7-Chlor-1-cyclopropyl-1,4-dihydro-4-oxo-1,8-naphthydridin-3-carbonsäure,
7-Chlor-1-(2,4-difluorphenyl)-1,4-dihydro-4-oxo-1,8-naphthyridin-3-carbonsäure.

Die als Ausgangsverbindungen verwendeten Amine der Formel (III) sind zum Teil bekannt. Chirale Amine können sowohl als Racemate, als auch als enantiomeren-oder diastereomerenreine Verbindungen eingesetzt werden.

Als Beispiele seien genannt:
2,7-Diazabicyclo[3.3.0]octan,
2,8-Diazabicyclo[4.3.0]nonan,

2-Methyl-2,8-diazabicyclo[4.3.0]nonan,
2-Oxa-5,8-diazabicyclo[4.3.0]nonan,
5-Methyl-2-oxa-5,8-diazabicyclo[4.3.0]nonan,
4-Amino-1,3,3a,4,7,7a-hexahydroisoindol,
4-Methylamino-1,3,3a,4,7,7a-hexahydroisoindol,
5-Methyl-4-methylamino-1,3,3a,4,7,7a-hexahydroisoindol,
7-Methyl-4-methylamino-1,3,3a,4,7,7a-hexahydroisoindol,
7a-Methyl-4-methylamino-1,3,3a,4,7,7a-hexahydroisoindol,
6,7-Dimethyl-4-methylamino-1,3,3a,4,7,7a-hexahydroisoindol,
4-Dimethylamino-1,3,3a,4,7,7a-hexahydroisoindol,
4-Ethylamino-1,3,3a,4,7,7a-hexahydroisoindol,
4-Aminoethyl-1,3,3a,4,7,7a-hexahydroisoindol,
4-Methylaminomethyl-1,3,3a,4,7,7a-hexahydroisoindol,
4-Hydroxy-1,3,3a,4,7,7a-hexahydroisoindol,
7-Isopropyl-4-methylamino-1,3,3a,4,7,7a-hexahydroisoindol,
4-Amino-7-isopropyl-1,3,3a,4,7,7a-hexahydroisoindol,
4-Hydroxymethyl-1,3,3a,4,7,7a-hexahydroisoindol,
4-Amino-7-cyclopropyl-1,3,3a,4,7,7a-hexahydroisoindol.

Die substituierten 1,3,3a,4,7,7a-Hexahydro-isoindole sind überwiegend neu. Sie können zum Beispiel durch Diels-Alder-Reaktion von Dienen der Formel (1)

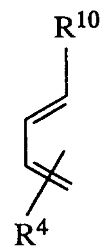

(1),

wobei $R^4$ die oben angegebene Bedeutung hat und $R^{10}$ entweder mit $R^3$ identisch ist oder eine funktionelle Gruppe darstellt, die in $R^3$ umgewandelt werden kann, mit Dienophilen der Formel (2),

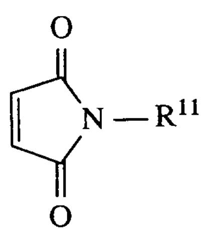

(2),

in welcher $R^{11}$ Wasserstoff oder eine Schutzgruppe wie Trimethylsilyl, Benzyl, $C_1$-$C_4$-Alkylphenylmethyl, Methoxybenzyl oder Benzylhydryl bedeuten, und der nachfolgenden Reduktion der Carbonylgruppen sowie gegebenenfalls der Abspaltung der Schutzgruppe erhalten werden.

Für die Diels-Alder-Reaktion kommen als Verdünnungsmittel alle inerten organischen Lösungsmittel in Frage. Hierzu gehören vorzugsweise Ether, wie Diisopropylether, Di-n-butylether, Dimethoxyethan, Tetrahydrofuran und Anisol, Kohlenwasserstoffe, wie z.B. Hexan, Methylcyclohexan, Toluol, Xylol und Mesitylen und halogenierte Kohlenwasserstoffe, wie z.B. Chloroform, 1,2-Dichlorethan und Chlorbenzol. Die Diels-Alder-Reaktion kann aber auch ohne Lösungsmittel durchgeführt werden.

Die Reaktionstemperaturen können in einem größeren Bereich variiert werden. Im allgemeinen arbeitet man zwischen etwa -20 °C und +200 °C, vorzugsweise zwischen -20 °C und +150 °C. Die Diels-Alder-Reaktion wird normalerweise bei Normaldruck durchgeführt. Zur Beschleunigung der Reaktion können aber auch Drucke bis zu 1,5 GPa eingesetzt werden.

Die Reduktion der Carbonylgruppen kann mit komplexen Hydriden erreicht werden. Als Hydride können z.B. Lithiumaluminiumhydrid, Lithiumborhydrid, Lithiumtriethylborhydrid, Natrium-bis-[2-methoxyethoxy]-aluminiumhydrid oder Natriumborhydrid in Gegenwart von Lewis-Säure-Katalysatoren wie Chlortrimethylsilan, Bortrifluorid-Etherat oder Aluminiumchlorid eingesetzt werden.

Als Verdünnungsmittel können Ether, wie z.B. Diethylether, Tetrahydrofuran, Dioxan oder Dimethoxyethan und Kohlenwasserstoffe, wie z.B. Hexan, Methylcyclohexan und Toluol oder auch deren Gemische verwendet werden.

Die Reaktionstemperaturen können im Bereich zwischen -40 und +180 °C, vorzugsweise zwischen 0 ° und 140 °C, variiert werden. Die Reduktion wird im allgemeinen unter Normaldruck durchgeführt, sie kann aber auch unter vermindertem Druck oder unter Überdruck durchgeführt werden.

Die Anwendung von Drucken zwischen 100 und 1000 kPa empfiehlt sich, um mit leicht siedenden Lösungsmitteln höhere Reaktionstemperaturen zu erreichen.

Die komplexen Hydride werden mindestens in einer der Stöchiometrie der Reduktion entsprechenden Menge eingesetzt. Im allgemeinen wird aber ein Überschuß, vorzugsweise zwischen 30 und 300 %, eingesetzt.

Die Abspaltung einer eventuellen Schutzgruppe erfolgt nach den allgemein bekannten Methoden der Schutzgruppenchemie (vergl. z.B. T.W. Greene, "Protective Groups in Organic Synthesis", John Wiley & Sons, New York 1981).

Die Ausgangsstoffe der Formeln (1) und (2) sind bekannt oder können nach allgemein bekannten Methoden der organischen Chemie hergestellt werden [vergl. z.B. J. Am. Chem. Soc. 100, 5179 (1978), J. Org. Chem. 43, 2164 (1978), DE 39 27 115, J. Org. Chem. 40, 24 (1975)].

Verwendet man beispielsweise 1-(tert.-Butyloxycarbonylamino)-1,3-butadien und Maleinimid als Ausgangsmaterialien und Lithiumaluminiumhydrid als Reduktionsmittel, so kann der Reaktionsablauf durch das folgende Formelschema wiedergegeben werden:

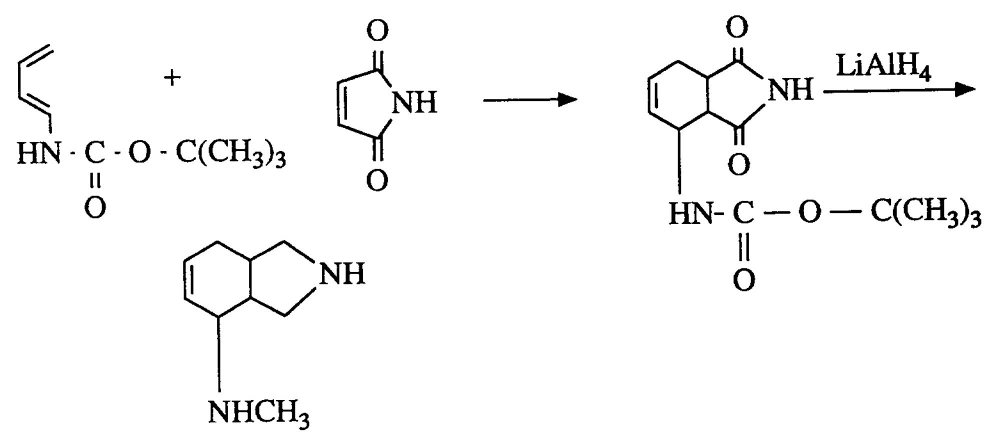


In einer besonderen Ausführungsform des Herstellungsverfahrens können alle Stufen bei Verwendung eines geeigneten Lösungsmittels, wie z.B. Tetrahydrofuran, ohne Isolierung der Zwischenprodukte durchgeführt werden. Verwendet man z.B. 1-(tert.-Butyloxycarbonylamino)-1,3-pentadien und N-Trimethylsilyl-maleinimid als Ausgangsmaterialien, so kann der Reaktionsablauf durch das folgende Formelschema wiedergegeben werden:

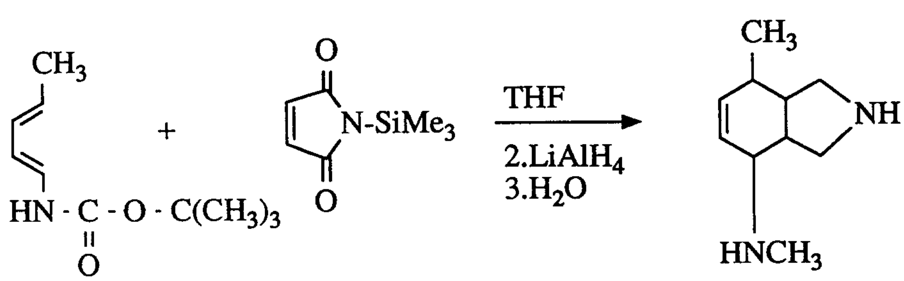


Durch NMR-Spektroskopie kann in diesem Fall nachgewiesen werden, daß alle Substituenten des Cyclohexen-Rings cis-Anordnung zueinander aufweisen.

Die Umsetzung von (II) mit (III), bei der die Verbindungen (III) auch in Form ihrer Salze, wie z.B. der Hydrochloride eingesetzt werden können, wird vorzugsweise in einem Verdünnungsmittel wie Dimethylsulfoxid, N,N-Dimethylformamid, N-Methylpyrrolidon, Hexamethyl-phosphorsäuretrisamid, Sulfolan, Acetonitril, Wasser, einem Alkohol wie Methanol, Ethanol, n-Propanol, Isopropanol, Glykolmonomethylether oder Pyridin vorgenommen. Ebenso können Gemische dieser Verdünnungsmittel verwendet werden.

Als Säurebinder können alle üblichen anorganischen und organischen Säurebindungsmittel verwendet werden. Hierzu gehören vorzugsweise die Alkalihydroxide, Alkalicarbonate, organische Amine und Amidine. Als besonders geeignet seien im einzelnen genannt: Triethylamin, 1,4-Diazabicyclo[2.2.2]octan (DABCO), 1,8-Diazabicyclo[5.4.0]undec-7-en (DBU) oder überschüssiges Amin (III).

Die Reaktionstemperaturen können in einem größeren Bereich variiert werden. Im allgemeinen arbeitet man zwischen etwa 20 und 200 °C, vorzugsweise zwischen 80 und 180 °C.

Die Umsetzung kann bei Normaldruck, aber auch bei erhöhtem Druck durchgeführt werden. Im allgemeinen arbeitet man bei Drucken zwischen etwa 1 und 100 bar, vorzugsweise zwischen 1 und 10 bar.

Bei der Durchführung des erfindungsgemäßen Verfahrens setzt man auf 1 Mol der Verbindung (II) 1 bis 15 Mol, vorzugsweise 1 bis 6 Mol der Verbindung (III) ein.

Freie Aminogruppen können während der Umsetzung durch eine geeignete Aminoschutzgruppe, zum Beispiel durch den tert.-Butoxycarbonylrest, geschützt und nach Beendigung der Reaktion durch Behandlung mit einer geeigneten Säure wie Chlorwasserstoffsäure oder Trifluoressigsäure wieder freigesetzt werden (siehe Houben-Weyl, Methoden der Organischen Chemie, Band E4, Seite 144 (1983); J.F.W. McOmie, Protective Groups in Organic Chemistry (1973), Seite 43).

Die erfindungsgemäßen Ester werden durch Umsetzung eines Alkalisalzes der zugrundeliegenden Carbonsäure, die gegebenenfalls am N-Atom durch eine Schutzgruppe wie den tert.-Butoxycarbonylrest geschützt sein kann, mit geeigneten Halogenalkylderivaten in einem Lösungsmittel wie Dimethylformamid, Dimethylacetamid, N-Methylpyrrolidon, Dimethylsulfoxid oder Tetramethylharnstoff bei Temperaturen von etwa 0 bis 100 °C, vorzugsweise 0 bis 50 °C, erhalten.

Die Herstellung der Säureadditionssalze der erfindungsgemäßen Verbindungen erfolgt in üblicher Weise zum Beispiel durch Lösen des Betains in ausreichender Menge wäßriger Säure und Ausfällen des Salzes mit einem mit Wasser mischbaren organischen Lösungsmittel wie Methanol, Ethanol, Aceton, Acetonitril. Man kann auch äquivalente Mengen Betain und Säure in Wasser oder einem Alkohol wie Glykolmonomethylether erhitzen und anschließend bis zur Trockne eindampfen oder das ausgefallene Salz absaugen. Als pharmazeutisch verwendbare Salze sind beispielsweise die Salze der Salzsäure, Schwefelsäure, Essigsäure, Glykolsäure, Milchsäure, Bernsteinsäure, Zitronensäure, Weinsäure, Methansulfonsäure, 4-Toluolsulfonsäure, Galacturonsäure, Gluconsäure, Embonsäure, Glutaminsäure oder Asparaginsäure zu verstehen.

Die Alkali- oder Erdalkalisalze der erfindungsgemäßen Carbonsäuren werden beispielsweise durch Lösen des Betains in unterschüssiger Alkali- oder Erdalkalilauge, Filtration von ungelöstem Betain und Eindampfen des Filtrats bis zur Trockne erhalten. Pharmazeutisch geeignet sind Natrium-, Kalium- oder Calciumsalze. Durch Umsetzung eines Alkali- oder Erdalkalisalzes mit einem geeigneten Silbersalz wie Silbernitrat werden die entsprechenden Silbersalze erhalten.

Außer den in den Beispielen genannten erfindungsgemäßen Verbindungen können auch die in der folgenden Tabelle aufgeführten hergestellt werden, die sowohl in racemischer als auch in enantiomerenreiner oder diastereomerenreiner Form vorliegen können:

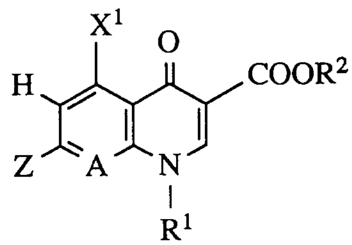

| $R^1$ | $R^2$ | $X^1$ | Z | A |
|---|---|---|---|---|
| Cyclopropyl | H | H | 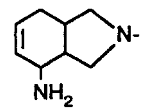 | C-H |
| Cyclopropyl | Ethyl | H | 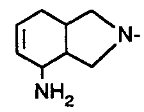 | C-H |
| Cyclopropyl | H | H | 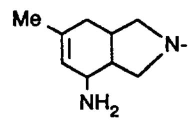 | C-Cl |
| Cyclopropyl | H | H | 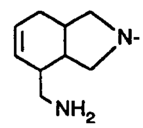 | C-Cl |
| Cyclopropyl | Ethyl | H | 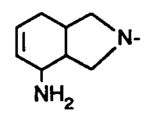 | C-Cl |
| Cyclopropyl | $-CH_2-CH_2-NH_2$ | H | 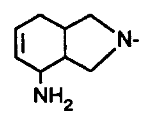 | C-Cl |
| Cyclopropyl | $-CH_2-CH_2-OCH_3$ | H | 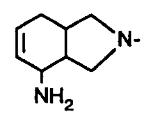 | C-Cl |
| Cyclopropyl | H | H | 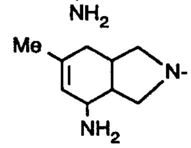 | C-F |
| Cyclopropyl | H | H | 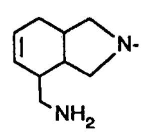 | C-F |

(Fortsetzung)

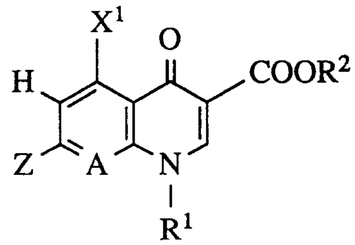

| $R^1$ | $R^2$ | $X^1$ | Z | A |
|---|---|---|---|---|
| Cyclopropyl | Ethyl | H | 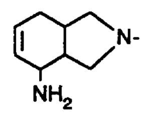 | C-F |
| Cyclopropyl | $-CH_2-CH_2-NH_2$ | H | 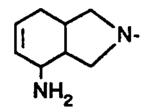 | C-F |
| Cyclopropyl | $-CH_2-CH_2-OCH_3$ | H | 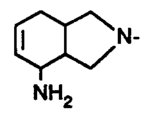 | C-F |
| Cyclopropyl | H | F | 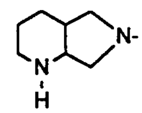 | C-F |
| Cyclopropyl | Ethyl | F | 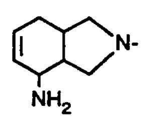 | C-F |
| Cyclopropyl | H | $NH_2$ | 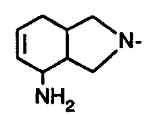 | C-F |
| Cyclopropyl | H | $-CF_3$ | 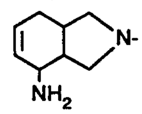 | C-F |
| Ethyl | H | H | 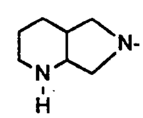 | C-Cl |

(Fortsetzung)

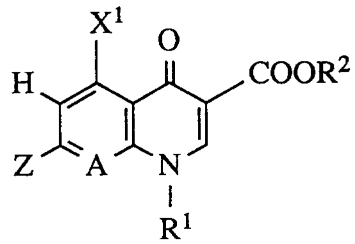

| $R^1$ | $R^2$ | $X^1$ | Z | A |
|---|---|---|---|---|
| Ethyl | Ethyl | H | 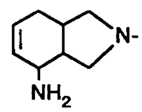 | C-Cl |
| 2,4-Difluorphenyl | H | H | 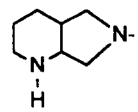 | C-Cl |
| 2,4-Difluorphenyl | Ethyl | H | 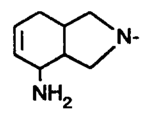 | C-Cl |
| Ethyl | H | H | 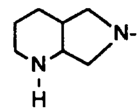 | C-F |
| Ethyl | H | H | 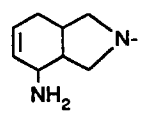 | C-F |
| Ethyl | Ethyl | H | 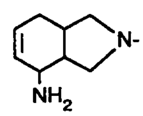 | C-F |
| 2,4-Difluorphenyl | H | H | 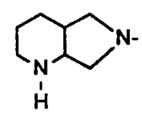 | C-F |
| 2,4-Difluorphenyl | H | H | 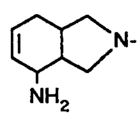 | C-F |
| 2,4-Difluorphenyl | Ethyl | H | 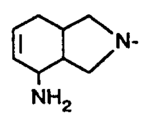 | C-F |

(Fortsetzung)

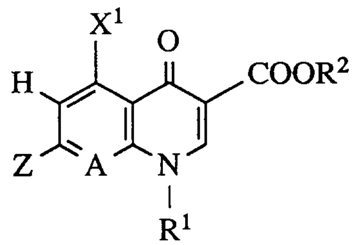

| $R^1$ | $R^2$ | $X^1$ | Z | A |
|---|---|---|---|---|
| Cyclopropyl | H | H | 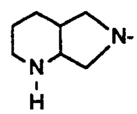 | C-$CH_3$ |
| Cyclopropyl | H | H | 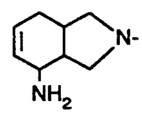 | C-$CH_3$ |
| Cyclopropyl | H | H | 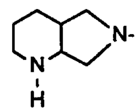 | N |
| Cyclopropyl | H | H | 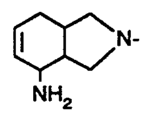 | N |
| Cyclopropyl | Ethyl | H | 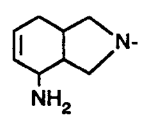 | N |
| 2,4-Difluorphenyl | H | H | 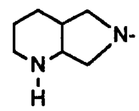 | N |
| 2,4-Difluorphenyl | H | H | 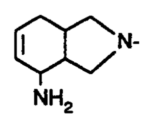 | N |
| 2,4-Difluorphenyl | Ethyl | H | 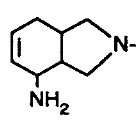 | N |
| 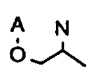 | H | H | 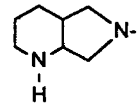 | siehe $R^1$ |

(Fortsetzung)

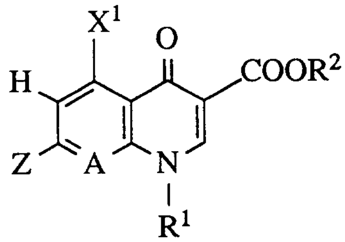

| $R^1$ | $R^2$ | $X^1$ | Z | A |
|---|---|---|---|---|
| 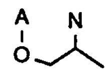 | H | H | 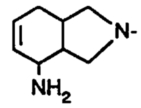 | siehe $R^1$ |
| 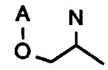 | Ethyl | H | 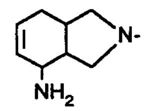 | siehe $R^1$ |
| Cyclopropyl | H | Br | 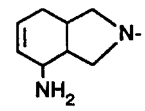 | C-F |
| Cyclopropyl | H | Cl | 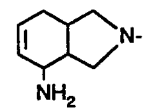 | C-F |
| Cyclopropyl | Ethyl | Br | 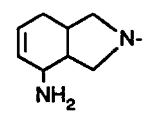 | C-Cl |
| Cyclopropyl | H | Cl | 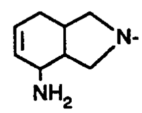 | C-Cl |
| Cyclopropyl | H | H | 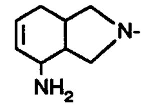 | $C-CH=CH_2$ |
| Cyclopropyl | H | H | 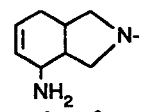 | $C-C\equiv CH$ |
| Cyclopropyl | H | H | 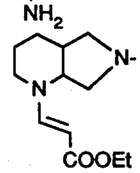 | C-Cl |
| Cyclopropyl | H | H | 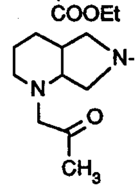 | C-Cl |

Die erfindungsgemäßen Verbindungen wirken stark antibiotisch und zeigen bei geringer Toxizität ein breites antibakterielles Spektrum gegen grampositive und gramnegative Keime, insbesondere gegen Enterobakterien; vor allem auch gegen solche, die resistent sind gegen verschiedene Antibiotika, wie z.B. Penicilline, Cephalosporine, Aminoglykoside, Sulfonamide, Tetracycline.

Diese wertvollen Eigenschaften ermöglichen ihre Verwendung als chemotherapeutische Wirkstoffe in der Medizin sowie als Stoffe zur Konservierung von anorganischen und organischen Materialien, insbesondere von organischen Materialien aller Art, z.B. Polymeren, Schmiermitteln, Farben, Fasern, Leder, Papier

und Holz, von Lebensmitteln und von Wasser.

Die erfindungsgemäßen Verbindungen sind gegen ein sehr breites Spektrum von Mikroorganismen wirksam. Mit ihrer Hilfe können gramnegative und grampositive Bakterien und bakterienähnliche Mikroorganismen bekämpft sowie die durch diese Erreger hervorgerufenen Erkrankungen verhindert, gebessert und/oder geheilt werden.

Die erfindungsgemäßen Verbindungen zeichnen sich durch verstärkte Wirkung auf ruhende und resistente Keime aus. Bei ruhenden Bakterien, also Bakterien, die kein nachweisbares Wachstum zeigen, wirken die Verbindungen weit unterhalb von Konzentrationen bisher bekannter Substanzen. Dies bezieht sich nicht nur auf die einzusetzende Menge, sondern auch auf die Geschwindigkeit der Abtötung. Solche Ergebnisse konnten bei grampositiven und -negativen Bakterien, insbesondere bei Staphylococcus aureus, Pseudomonas aeruginosa, Enterococcus faecalis und Escherichia coli beobachtet werden.

Auch gegenüber Bakterien, die gegenüber vergleichbaren Substanzen als weniger empfindlich eingestuft werden, insbesondere resistenten Staphylococcus aureus, Escherichia coli, Pseudomonas aeruginosa und Enterococcus faecalis zeigen die erfindungsgemäßen Verbindungen überraschende Wirkungssteigerungen.

Besonders wirksam sind die erfindungsgemäßen Verbindungen gegen Bakterien und bakterienähnliche Mikroorganismen. Sie sind daher besonders gut zur Prophylaxe und Chemotherapie von lokalen und systemischen Infektionen in der Human-und Tiermedizin geeignet, die durch diese Erreger hervorgerufen werden.

Die Verbindungen eignen sich ferner zur Bekämpfung von Protozoonosen und Helminthosen.

Die erfindungsgemäßen Verbindungen können in verschiedenen pharmazeutischen Zubereitungen angewendet werden. Als bevorzugte pharmazeutische Zubereitungen seien Tabletten, Dragees, Kapseln, Pillen, Granulate, Suppositorien, Lösungen, Suspensionen und Emulsionen, Pasten, Salben, Gele, Cremes, Lotions, Puder und Sprays genannt.

Die minimalen Hemmkonzentrationen (MHK) wurden per Reihenverdünnungsverfahren auf Iso-Sensitest Agar (Oxoid) bestimmt. Für jede Prüfungssubstanz wurde eine Reihe von Agarplatten hergestellt, die bei jeweils doppelter Verdünnung abfallende Konzentrationen des Wirkstoffs enthielten. Die Agarplatten wurden mit einem Multipoint-Inokulator (Denley) beimpft. Zum Beimpfen wurden Übernachtkulturen der Erreger verwandt, die zuvor so verdünnt wurden, daß jeder Impfpunkt ca. $10^4$ koloniebildende Partikel enthielt. Die beimpften Agarplatten wurden bei 37 °C bebrütet, und das Keimwachstum wurde nach ca. 20 Stunden abgelesen. Der MHK-Wert (µg/ml) gibt die niedrigste Wirkstoffkonzentration an, bei der mit bloßem Auge kein Wachstum zu erkennen war.

In der nachstehenden Tabelle sind die MHK-Werte einiger der erfindungsgemäßen Verbindungen im Vergleich zu 7-(3-Aminopyrrolidin-1-yl)-1-cyclopropyl-8-fluor-1,4-dihydro-4-oxo-3-chinolincarbonsäure (Journal of Medicinal Chemistry 35, 198 (1992)) als Referenzverbindung aufgeführt.

Tabelle

| MHK-Werte | | | | | | |
|---|---|---|---|---|---|---|
| Spezies | Stamm | Beispiel Nr. | | | | Referenz |
| | | 7 | 8 | 20 | 21 | |
| E. coli | Neumann | 0,0078 | 0,015 | 0,03 | 0,015 | 0,031 |
| | 455/7 | 0,5 | 0,5 | 1 | 0,5 | 1 |
| Klebsiella pneumoniae | 8085 | 0,015 | 0,03 | 0,03 | 0,3 | 0,062 |
| | 63 | 0,015 | 0,03 | 0,03 | 0,3 | 0,062 |
| Providencia sp. | 12012 | 0,015 | 0,06 | 0,06 | 0,06 | 0,06 |
| | 12052 | 1 | 1 | 1 | 1 | 2 |
| Mirococcus luteus | 9341 | 0,03 | 0,06 | 0,06 | 0,125 | 0,5 |
| Staphylococcus aureus | ICB 25701 | 0,06 | 0,06 | 0,25 | 0,25 | 4 |
| | 1756 | 0,0039 | 0,0078 | 0,015 | 0,015 | 0,125 |
| | 133 | 0,0039 | 0,0078 | 0,03 | 0,015 | 0,125 |
| | 25768 | 2 | 2 | 4 | 2 | 16 |
| Enterococcus faecalis | 27101 | 0,015 | 0,06 | 0,06 | 0,06 | 0,25 |
| | 9790 | 0,03 | 0,06 | 0,06 | 0,06 | 0,25 |
| Acinetobacter caloaceticus | 14068 | 0,015 | 0,03 | 0,06 | 0,03 | 0,25 |

Herstellung der Zwischenprodukte

Beispiel A

4-Methylamino-1,3,3a,4,7,7a-hexahydroisoindol

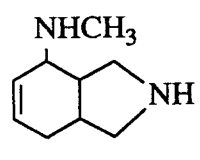

Methode I:

14,4 g (60 mmol) 70 %iges 1-(tert.-Butyloxycarbonylamino)-1,3-butadien [J.Org.Chem. 43, 2164 (1978)] werden als Lösung in 30 ml absolutem Tetrahydrofuran zu vorgelegten 10,1 g (60 mmol) N-Trimethylsilyl-maleinimid [J.Org.Chem. 40, 24 (1975)] in 30 ml absolutem Tetrahydrofuran getropft. Nach dem Abklingen der exothermen Reaktion wird noch 1 Stunde unter Rückflußkühlung gekocht.

Die erkaltete Reaktionsmischung wird dann unter Stickstoff zu vorgelegten 7,6 g (0,2 mol) Lithiumaluminiumhydrid in 200 ml absolutem Tetrahydrofuran getropft. Dann wird 14 Stunden unter Rückflußkühlung gekocht. Zu der erkalteten Reaktionsmischung werden dann nacheinander 7,6 g Wasser in 23 ml Tetrahydrofuran, 7,6 g 10 %ige Natronlauge und 22,8 g Wasser getropft. Die Salze werden abfiltriert und das Filtrat im Vakuum eingeengt. Der Rückstand (10,3 g) wird bei 87 °C/0,8 mbar destilliert.

Das Destillat wird in 80 ml absolutem Pentan aufgenommen, filtriert und das Produkt durch Abkühlen auf -70 °C kristallisiert.

Ausbeute: 3,3 g, Schmelzpunkt: 72-82 ° C.

Durch Behandeln mit äquimolarer Menge 2n Salzsäure erhält man 4-Methylamino-1,3,3a,4,7,7a-hexahydro-isoindol-Dihydrochlorid vom Schmelzpunkt 265-268 ° C (aus Methanol).

Methode II:

a) 4-(tert.-Butyloxycarbonylamino)-1,3-dioxo-1,3,3a,4,7,7a-hexahydroisoindol,
48,8 g (0,5 mol) Maleinimid werden in 200 ml absolutem Tetrahydrofuran gelöst vorgelegt und 120 g (0,5 mol) ca 70 %iges 1-(tert.-Butyloxycarbonylamino)-1,3-butadien als Lösung in 500 ml absolutem Tetrahydrofuran zugetropft, wobei die Temperatur bei 20 bis 30 ° C gehalten wird. Über Nacht wird bei Raumtemperatur nachgerührt. Dann wird eingeengt und aus Essigsäureethylester umkristallisiert. Es werden 57 g Produkt mit einem Schmelzpunkt von 177 bis 182 ° C erhalten. Aus der Mutterlauge werden weitere 13 g mit einem Schmelzpunkt von 158 bis 160 ° C erhalten.
b) 4-Methylamino-1,3,3a,4,7,7a-hexahydroisoindol,
27,1 g (0,71 mol) Lithiumaluminiumhydrid werden unter Stickstoff in 300 ml absolutem Tetrahydrofuran vorgelegt und eine Lösung von 57 g (0,21 mol) 4-(tert.-Butyloxycarbonylamino)-1,3-dioxo-1,3,3a,4,7,7a-hexahydroisoindol in 570 ml absolutem Tetrahydrofuran zugetropft. Dann wird über Nacht unter Rückflußkühlung gekocht. Nach dem Erkalten werden nacheinander, 27,1 g Wasser in 82 ml Tetrahydrofuran, 27,1 g 10 %ige Natronlauge und 81,3 g Wasser zu dem Ansatz getropft. Die Salze werden abgesaugt, mit Tetrahydrofuran gewaschen und das Filtrat im Vakuum eingeengt. Der Rückstand wird im Hochvakuum destilliert.
Ausbeute: 19,1 g

Beispiel B

4-Amino-1,3,3a,4,7,7a-hexahydroisoindol

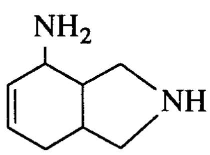

13,3 g (50 mmol) 4-tert.-Butyloxycarbonylamino-1,3-dioxo-1,3,3a,4,7,7a-hexahydroisoindol (aus Beispiel A, Methode II) werden in 166 ml Trifluoressigsäure über Nacht bei Raumtemperatur gerührt. Dann wird die Trifluoressigsäure bei 10 mbar abdestilliert und der Rückstand bei 50 ° im Hochvakuum von Säureresten befreit Anschließend wird in absolutem Tetrahydrofuran aufgenommen und im Vakuum eingeengt. Der Rückstand wird in 100 ml absolutem Tetrahydrofuran aufgenommen und unter Stickstofff zu einer Lösung von 11,3 g (0,3 mol) Lithiumaluminiumhydrid in 300 ml absolutem Tetrahydrofuran getropft. Dann wird 16 Stunden unter Rückflußkühlung gekocht Nach dem Erkalten werden nacheinander 11,3 g Wasser in 34 ml Tetrahydrofuran, 11,3 ml 10 %ige Natronlauge und 34 ml Wasser zugetropft. Der Niederschlag wird abgesaugt und mit Tetrahydrofuran gewaschen. Das Filtrat wird eingeengt und der Rückstand destilliert.
Ausbeute: 2,2 g, Gehalt: 92 % (gaschromatographisch bestimmt)
Siedepunkt: 70 ° /0,2 mbar

Beispiel C

7-Methyl-4-methylamino-1,3,3a,4,7,7a-hexahydroisoindol

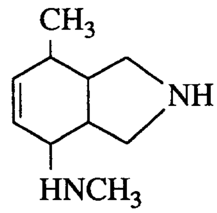

In Analogie zu Beispiel A, Methode I werden 21,9 g (0,12 mol) 1-(tert.-Butyloxycarbonylamino)-1,3-pentadien mit 20,3 g (0,12 mol) N-Trimethylsilyl-maleinimid umgesetzt und anschließend mit 15,2 g (0,4 mol) Lithiumaluminiumhydrid reduziert. Das Rohprodukt wird aus Tetrahydrofuran umkristallisiert.
Ausbeute: 6,2 g, Schmelzpunkt: 106-108 ° C.

Beispiel D

7-Isopropyl-4-methylamino-1,3,3a,4,7,7a-hexahydroisoindol

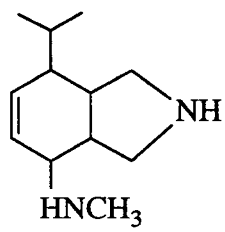

50 g (0,24 mol) 1-(tert-Butyloxycarbonylamino)-5-methyl-1,3-hexadien werden zusammen mit 23 g (0,24 mol) Maleinimid in 75 ml Ethanol und 75 ml Wasser 24 Stunden unter Rückfluß gerührt. Nach dem Abkühlen saugt man den Feststoff ab, wäscht mit Wasser nach und erhält nach dem Trocknen 56,3 g (76 % der Theorie) eines Feststoffes mit dem Schmelzpunkt 192-195 ° C. 15 g (0,049 mol) werden zusammen mit 11 g (0,29 mol) Lithiumaluminiumhydrid in 300 ml Tetrahydrofuran 10 Stunden unter Rückfluß gerührt. Nach dem Abkühlen wird mit 10 ml Wasser hydrolysiert. Man saugt von Niederschlag ab, wäscht mit Tetrahydrofuran nach, und engt die vereinigten Filtrate zur Trockne ein. Es werden 8,7 g eines Feststoffes erhalten, der durch Kristallisation (Petrolether-Essigsäureethylester = 1:5) gereinigt wird.
Ausbeute: 43,5 g (51 % der Theorie),
Schmelzpunkt: 76-81 ° C.

Beispiel E

4-Amino-7-isopropyl-1,3,3a,4,7,7a-hexahydroisoindol

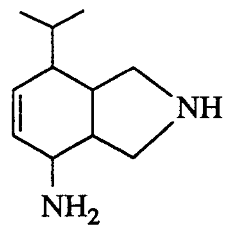

Die Darstellung erfolgt analog Beispiel B.

$^1$H-NMR (200 MHz, $CDCl_3$): δ = 0,95 (6H); 2,3-2,7 (m, 7H); 5,75 (2H). MS: m/e (% rel.Int.): 180 [$M^+$] (7); 163 (45); 120 (100); 67 (100).

Beispiel F

4-Hydroxymethyl-1,3,3a,4,7,7a-hexahydroisoindol

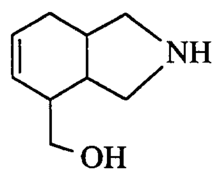

25 g (0,22 mol) 2,4-Pentadiencarbonsäuremethylester werden in 100 ml Dioxan mit 20 g (0,21 mol) Maleinsäureimid 40 Stunden unter Rückfluß gerührt. Das nach dem Einengen erhaltene Öl (51 g) wird in 350 ml Tetrahydrofuran mit 20 g (0,52 mol) Lithiumaluminiumhydrid 16 Stunden unter Rückfluß gerührt. Nach dem Abkühlen wird mit 63 ml Wasser, 63 ml 10 proz. Natronlauge und zuletzt 60 ml Wasser hydrolysiert, vom Niederschlag abgesaugt und dieser noch mehrmals mit Tetrahydrofuran nachgewaschen. Die vereinigten Filtrate werden eingeengt und im Hochvakuum destilliert.
Ausbeute: 10 g (30 % der Theorie)
Siedepunkt: 96-115 °C/0,07 mbar.

Beispiel G

4-Methylaminomethyl-1,3,3a,4,7,7a-hexahydroisoindol

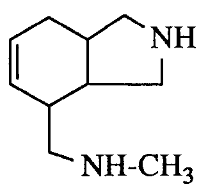

a) 1-tert.-Butyloxycarbonylamino-2,4-pentadien

Durch Umsetzung von 1-Amino-2,4-pentadien (P.A. Grieco et al., Tetrahedron 42, 2847 [1986]) mit Di-tert.-butylcarbonat in Dioxan bei Raumtemperatur während 12 Stunden bei pH 8-10 erhält man 1-tert.-Butyloxycarbonylamino-2,4-pentadien als helles Öl in quantitativer Ausbeute.

$^1$H-NMR (200 MHz, $CDCl_3$): δ = 1,45 (9H), 3,78 (2H); 4,65 (br., 1H); 5,05-5,21 (m, 2H); 5,60-5,75 (m, 1H); 6,08-6,42 ppm (m, 2H).

b) 4-tert.-Butyloxycarbonylaminomethyl-1,3-dioxo-1,3,3a,4,7,7a-hexahydroisoindol,
30 g (0,16 mol) 1-tert-Butyloxycarbonylamino-2,4-pentadien werden zusammen mit 16 g (0,16 mol) Maleinimid in 120 ml Dioxan 12 Stunden bei Rückfluß gerührt. Nach dem Abkühlen engt man zur Hälfte ein und saugt den Feststoff ab.
Ausbeute: 35,3 g (76 %)
Schmelzpunkt: 197,5-198,5 ° C.
c) 4-Methylaminomethyl-1,3,3a,4,7,7a-hexahydroisoindol,
Man reduziert 4-tert.-Butyloxycarbonylaminomethyl-1,3-dioxo-1,3,3a,4,7,7a-hexahydroisoindol mit Lithiumaluminiumhydrid analog wie in Beispiel A, Methode IIb beschrieben: gelbes Öl.
Siedepunkt = 78 ° C/0,05 mbar.

Beispiel H

4-Aminomethyl-1,3,3a,4,7,7a-hexahydroisoindol

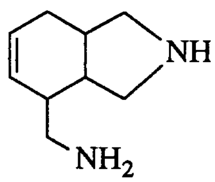

Man setzt 4-tert.-Butyloxycarbonylaminomethyl-1,3-dioxo-1,3,3a,4,7,7a-hexahydroisoindol analog wie in Beispiel B beschrieben ein.
Siedepunkt = 135-140 ° C/0,1 mbar.

Beispiel I

6-Methyl-4-methylamino-1,3,3a,4,7,7a-hexahydroisoindol

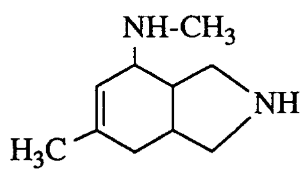

a) 4-(tert.-Butyloxycarbonylamino)-1,3-dioxo-6-methyl-1,3,3a,4,7,7a-hexahydroisoindol,
Entsprechend Beispiel A/Methode IIa setzt man mit 1-tert-Butyloxycarbonylamino-3-methyl-1,3-butadien in Dioxan um. Schmelzpunkt: 135 ° C
b) 6-Methyl-4-methylamino-1,3,3a,4,7,7a-hexahydroisoindol,
In Analogie zu Beispiel B werden 5,6 g (20 mmol) des Produkts aus Beispiel Ma) mit 2,2 g (60 mmol) Lithiumaluminiumhydrid in 60 ml Tetrahydrofuran 15 Stunden unter Rückfluß erhitzt. Bei der destillativen Aufarbeitung werden 1,2 g des Produkts vom Siedepunkt 68-71 ° C/0,2-0,3 mbar erhalten.

Beispiel J

4-Amino-7-methyl-1,3,3a,4,7,7a-hexahydroisoindol

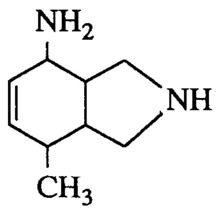

a) 4-(tert.-Butyloxycarbonylamino)-1,3-dioxo-7-methyl-1,3,3a,4,7,7a-hexahydroisoindol,
Entsprechend Beispiel A/Methode IIa setzt man mit 1-tert-Butyloxycarbonylamino-1,3-pentadien um und kristallisiert das Reaktionsprodukt aus Dioxan um.
Ausbeute: 79 %
Schmelzpunkt: 298-211 ° C
b) 4-Amino-7-methyl-1,3,3a,4,7,7a-hexahydroisoindol,
Entsprechend Beispiel B setzt man das Produkt aus Beispiel Na) ein und erhält das freie Amin als Öl vom Siedepunkt 83-92 ° C/0,1 mbar, das beim Stehen kristallisiert.
Gehalt: 90 %ig (nach Gaschromatogramm)

Beispiel K

4-Amino-7-cyclopropyl-1,3,3a,4,7,7a-hexahydroisoindol

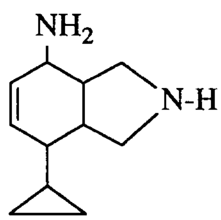

a) 4-(tert.-Butoxycarbonylamino)-7-cyclopropyl-1,3-dioxo-1,3,3a,4,7,7a-hexahydroisoindol
1-tert.-Butoxycarbonylamino-4-cyclopropyl-1,3.-butadien (hergestellt analog der in J. Org, Chem. 43, 2164 [1978] beschriebenen Methode; IR ($CCl_4$): 330, 1720, 1605 $cm^{-1}$) wird analog Beispiel A/Methode II umgesetzt.
Schmelzpunkt: 195,5-196,5 ° C.
b) 4-Amino-7-cyclopropyl-1,3,3a,4,7,7a-hexahydroisoindol
Das Produkt aus Beispiel Oa wird analog Beispiel B mit Lithiumaluminiumhydrid zu einem viskosen Öl umgesetzt.
FAB-MS (Glycerin/DMSO): m/e 179 $[M+H]^+$).

Beispiel L

1-Cyclopropyl-7-fluor-1,4-dihydro-4-oxo-3-chinolincarbonsäure

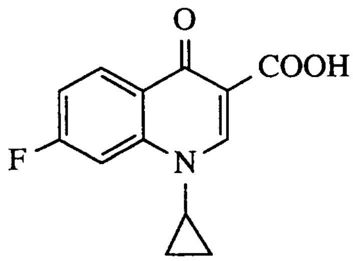

a) (2,4-Difluorbenzoyl)malonsäurediethylester
Man legt 13,47 g (0,14 mol) Magnesiumchlorid bei 0 °C in 140 ml absolutem Acetonitril vor und tropft unter Eisbadkühlung 23,5 g (0,14 mol) Malonsäurediethylester zu. Anschließend werden 28,17 g (0,28 mol) Triethylamin bei 0 °C zugetropft, nach 30 minütigem Nachrühren werden bei 0 °C 22,4 g (0,14 mol) 2,4-Difluorbenzoesäurefluorid (US 4.847.442) zugetropft und unter Erwärmung auf Raumtemperatur über Nacht nachgerührt. Es wird mit 90 ml 18 %iger Salzsäure versetzt und mit Methylenchlorid extrahiert, über Natriumsulfat getrocknet und im Vakuum eingeengt.
Rohausbeute: 41,4 g
b) (2,4-Difluorbenzoyl)essigsäureethylester
41,4 g des rohen (2,4-Difluorbenzoyl)malonsäurediethylesters werden in 130 ml Wasser mit 170 mg p-Toluolsulfonsäure 8,5 Stunden zum Rückfluß erhitzt. Es wird mit Methylenchlorid extrahiert, mit Wasser gewaschen, über Natriumsulfat getrocknet und im Vakuum eingeengt.
Rohausbeute: 29,0 g
c) 2-(2,4-Difluorbenzoyl)-3-ethoxyacrylsäureethylester
29,0 g (0,127 mol) des Produktes von b. werden in 29,98 g (0,20 mol) Orthoameisensäureethylester und 34,5 g (0,34 mol) Essigsäureanhydrid zwei Stunden auf 150-160 °C erwärmt. Alle leicht flüchtigen Bestandteile werden im Hochvakuum bei einer Badtemperatur bis 100 °C abdestilliert und das Rohprodukt direkt weiter umsetzt.
Rohausbeute: 28,4 g
d) 3-Cyclopropylamino-2-(2,4-difluorbenzoyl)acrylsäureethylester
Zu 28,4 g des Rohproduktes von c. werden in 220 ml Ethanol bei 0 °C 6,27 g (0,11 mol) Cyclopropylamin getropft und zwei Stunden bei Raumtemperatur nachgerührt. Es wird mit 220 im Wasser versetzt und das auskristallisierte Produkt isoliert.
Ausbeute: 22,2 g (54 % der Theorie)
Schmelzpunkt: 71 °C
e) 1-Cyclopropyl-7-fluor-1,4-dihydro-4-oxo-3-chinolin-carbonsäureethylester
2,95 g (0,01 mol) des unter d. erhaltenen Produktes werden in 33 ml Dimethylformamid mit 0,63 g (0,015 mol) Natriumfluorid sechs Stunden auf 140 °C erwärmt. Nach dem Abkühlen wird mit Wasser versetzt und das auskristallisierte Produkt isoliert und bei 100 °C getrocknet.
Ausbeute: 2,1 g (76 % der Theorie)
Schmelzpunkt: 190-192 °C
f) 1-Cyclopropyl-7-fluor-1,4-dihydro-4-oxo-3-chinolincarbonsäure
2,75 g (0,01 mol) 1-Cyclopropyl-7-fluor-1,4-dihydro-4-oxo-3-chinolincarbonsäureethylester werden in einer Mischung aus 12 ml Essigsäure, 12 ml Wasser und 1,2 ml konzentrierter Schwefelsäure vier Stunden unter Rückfluß erhitzt. Das abgekühlte Reaktionsgemisch wird auf Eiswasser gegeben, der Niederschlag abfiltriert, mit Wasser nachgewaschen und im Trockenschrank bei 100 °C getrocknet.
Ausbeute: 2,2 g (89 % der Theorie)
Schmelzpunkt: 291 °C (Zersetzung)

Beispiel M

8-Chlor-1-cyclopropyl-7-fluor-1,4-dihydro-4-oxo-3-chinolincarbonsäure

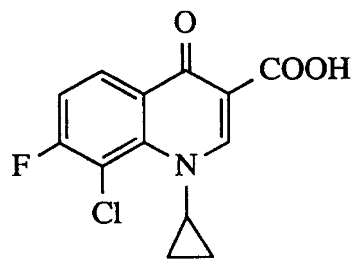

a) 3-Chlor-2,4-difluorbenzoesäure
Zu 400 ml konzentrierter Schwefelsäure werden 200 g (0,92 mol) 3-Chlor-2,4-difluorbenzotrifluorid gegeben und für drei Stunden unter Rühren auf 118 °C erhitzt. Nach dem Abkühlen wird auf 500 g Eis ausgetragen, der weiße Feststoff abgesaugt und im Vakuum bei 60 °C getrocknet.
Ausbeute: 172 g (97 % der Theorie)
Schmelzpunkt: 173-175 °C
b) 3-Chlor-2,4-difluor-benzoesäurechlorid
Zu einer Suspension von 235 g (1,22 mol) 3-Chlor-2,4-difluorbenzoesäure in 800 ml Toluol und 3 ml Dimethylformamid wird bei 70 °C so lange Thionylchlorid dosiert, bis eine klare Lösung entstanden ist und keine Gasentwicklung mehr beobachtet wird. Danach wird das Toluol und überschüssiges Thionylchlorid abdestilliert und anschließend das Produkt durch Destillation erhalten.
Ausbeute: 256 g (99 % der Theorie)
Siedepunkt: 108-110 °C/22 mbar
c) (3-Chlor-2,4-difluorbenzoyl)malonsäurediethylester
3,9 g (0,16 mol) Magnesium werden in 8,6 ml Ethanol vorgelegt und die Reaktion mit Tetrachlorkohlenstoff gestartet. Bei 50-60 °C Innentemperatur wird eine Lösung von 23,1 g (0,144 mol) Malonsäurediethylester in 16,3 ml Ethanol so zugetropft, daß die Temperatur gehalten wird. Anschließend wird eine Stunde bei 60 °C nachgerührt. Danach wird bei -10 bis -5 °C eine Lösung von 31,3 g (0,148 mol) 3-Chlor-2,4-difluorbenzoesäurechlorid in 16 ml Toluol zugetropft, eine Stunde bei 0 °C und anschließend unter Erwärmung auf Raumtemperatur über Nacht nachgerührt. Das Reaktionsgemisch wird auf Eiswasser gegeben, mit 10 ml konzentrierter Schwefelsäure angesäuert und mit Toluol extrahiert. Es wird mit gesättigter Natriumchloridlösung gewaschen und das Lösungsmittel im Vakuum entfernt.
Rohausbeute: 49,9 g
d) (3-Chlor-2,4-difluorbenzoyl)essigsäureethylester
49,9 g des unter c. erhaltenen Rohproduktes werden in 60 ml Wasser mit 1,83 g p-Toluolsulfonsäure 4,5 Stunden zum Rückfluß erhitzt. Der erkaltete Ansatz wird mit Methylenchlorid extrahiert, mit gesättigter Natriumchloridlösung gewaschen, über Natriumsulfat getrocknet und im Vakuum eingeengt.
Rohausbeute: 37,3 g
e) 2-(3-Chlor-2,4-difluorbenzoyl)-3-ethoxyacrylsäureethylester
37,3 g des unter d. erhaltenen Rohproduktes werden mit 33,4 g (0,226 mol) Orthoameisensäureethylester und 37,2 g (0,365 mol) Essigsäureanhydrid zwei Stunden auf 150-160 °C erwärmt. Überschüssiges Reagenz wird zunächst im Vakuum und anschließend im Hochvakuum bis zu einer Badtemperatur von 100 °C entfernt.
Rohausbeute: 40,2 g
f) 2-(3-Chlor-2,4-difluorbenzoyl)-3-cyclopropylaminoacrylsäureethylester
40,2 g des unter e. erhaltenen Rohproduktes werden in 100 ml Ethanol gelöst und unter Eisbadkühlung 9,6 g (0,168 mol) Cyclopropylamin zugetropft. Es wird 30 Minuten bei Raumtemperatur nachgerührt und anschließend das Reaktionsgemisch mit 100 ml Eiswasser versetzt. Das ausgefallene Produkt wird isoliert, mit Wasser gewaschen und bei 100 °C getrocknet.
Ausbeute: 30,8 g (63 % der Theorie bezogen auf (c))
Schmelzpunkt: 101-104 °C
g) 8-Chlor-1-cyclopropyl-7-fluor-1,4-dihydro-4-oxo-3-chinolincarbonsäureethylester
15 g (0,046 mol) des unter f. erhaltenen Rohproduktes werden in 90 ml Dimethylformamid mit 7,2 g

(0,052 mol) Kaliumcarbonat zwei Stunden auf 140-150 ° C erwärmt. Der erkaltete Ansatz wird auf Wasser gegeben, das Produkt isoliert, mit Wasser nachgewaschen und bei 100 ° C getrocknet.
Ausbeute: 13,5 g (95 % der Theorie)
Schmelzpunkt: 149-153 ° C
h) 8-Chlor-1-cyclopropyl-7-fluor-1,4-dihydro-4-oxo-3-chinolincarbonsäure
13,5 g (0,044 mol) des unter g. erhaltenen Esters werden in einer Mischung aus 52 ml Essigsäure, 52 ml Wasser und 5,2 ml konzentrierter Schwefelsäure vier Stunden zum Rückfluß erhitzt. Der erkaltete Ansatz wird auf Eiswasser gegeben, das Produkt wird isoliert, gründlich mit Wasser nachgewaschen und bei 100 ° C getrocknet.
Ausbeute: 11,6 g (94 % der Theorie)
Schmelzpunkt: 192-193 ° C

Beispiel N

1-Cyclopropyl-5,7,8-trifluor-1,4-dihydro-4-oxo-3-chinolincarborsäure

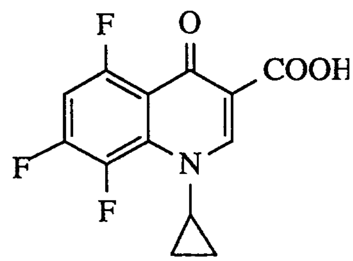

36,7 g (0,118 mol) 1-Cyclopropyl-5,7,8-trifluor-1,4-dihydro-4-oxo-3-chinolincarbonsäureethylester (JP 1308281) werden in eine Mischung aus 285 ml Essigsäure, 190 ml Wasser und 30 ml konzentrierter Schwefelsäure eingetragen und zwei Stunden zum Rückfluß erhitzt. Der Ansatz wird auf Eis gegeben, das Produkt isoliert, mit Wasser nachgewaschen und bei 100 ° C getrocknet.
Ausbeute: 31,6g (94 % der Theorie)
Schmelzpunkt: 218-220 ° C

Beispiel O

1-Ethyl-7,8-difluor-1,4-dihydro-4-oxo-3-chinolincarbonsäure

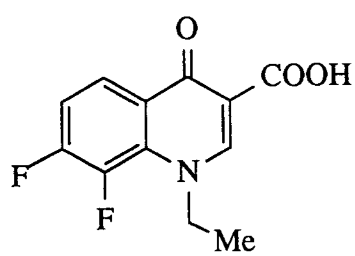

a) 2,3,4-Trifluorbenzoesäurechlorid
Zu 1300 ml Thionylchlorid und 5 ml Dimethylformamid werden bei 50 ° C portionsweise über eine Dosierschnecke 1000 g (5,68 mol) 2,3,4-Trifluorbenzoesäure eingetragen. Nach dem Ende der Dosierung wird so lange zum Rückfluß erhitzt bis die Gasentwicklung beendet ist. Danach wird das überschüssige Thionylchlorid durch Destillation abgetrennt und das Produkt im Vakuum destilliert.
Ausbeute: 1027 g (93 % der Theorie)
Siedepunkt: 65 ° C / 10 mbar

b) (2,3,4-Trifluorbenzoyl)malonsäurediethylester

3,6 g (0,148 mol) Magnesiumspäne werden in 8,1 ml Ethanol vorgelegt, die Reaktion mit einigen Tropfen Tetrachlorkohlenstoff gestartet und anschließend die Lösung von 21,8 g (0,136 mol) Malonsäurediethylester in 15 ml Ethanol und 58 ml Toluol so zugetropft, daß die Innentemperatur zwischen 50 und 60 ° C liegt. Anschließend wird eine Stunde bei 60 ° C nachgerührt. Bei -10 bis -5 ° C wird die Lösung von 27,6 g (0,15 mol) 2,3,4-Trifluorbenzoesäurechlorid in 15,4 ml Toluol zugetropft, eine Stunde bei 0 ° C und anschließend unter Erwärmung auf Raumtemperatur über Nacht nachgerührt. Der Ansatz wird auf 60 ml Eiswasser gegeben, mit 9,7 ml konzentrierter Schwefelsäure versetzt und mit Toluol extrahiert. Es wird mit gesättigter Natriumchloridlösung gewaschen und das Lösungsmittel im Vakuum entfernt.

Rohausbeute: 45,2 g

c) (2,3,4-Trifluorbenzoyl)essigsäureethylester

45,2 g des bei b. erhaltenen Rohproduktes werden in 57 ml Wasser mit 1,66 g p-Toluolsulfonsäure 4,5 Stunden zum Rückfluß erhitzt. Der erkaltete Ansatz wird mit Methylenchlorid extrahiert, mit gesättigter Natriumchloridlösung gewaschen, über Natriumsulfat getrocknet und im Vakuum eingeengt.

Rohausbeute: 33 g

d) 3-Ethoxy-2-(2,3,4-trifluorbenzoyl)acrylsäureethylester

33 g des unter c. erhaltenen Produktes werden mit 31,5 g (0,213 mol) Orthoameisensäureethylester und 31,5 g (0,344 mol) Essigsäureanhydrid zwei Stunden auf 150-160 ° C erwärmt. Überschüssiges Reagenz wird zunächst im Vakuum, dann im Hochvakuum bis zu einer Badtemperatur von 100 ° C entfernt.

Rohausbeute: 34,5 g

e) 3-Ethylamin-2-(2,3,4-trifluorbenzoyl)acrylsäureethylester

9,06 g (0,03 mol) des unter d. erhaltenen Produktes werden in 60 ml Ethanol bei 0 ° C vorgelegt und 2,12 ml (0,033 mol) einer 70 %igen Ethylaminlösung zugetropft. Es wird vier Stunden bei Raumtemperatur nachgerührt, 60 ml Wasser zugetropft und das ausfallende Produkt isoliert. Es wird mit Wasser nachgewaschen und bei ca. 100 ° C getrocknet.

Ausbeute: 5,0 g (55 % der Theorie)

Schmelzpunkt: 106-108 ° C

f) 1-Ethyl-7,8-difluor-1,4-dihydro-4-oxo-3-chinolincarbonsäureethylester

5,0 g (0,017 mol) des unter e. erhaltenen Produktes werden mit 2,6 g (0,019 mol) Kaliumcarbonat in 30 ml Dimethylformamid vier Stunden auf 100 ° C erwärmt. Der erkaltete Ansatz wird auf Eiswasser gegeben, das Produkt isoliert, mit Wasser nachgewaschen und bei 100 ° C getrocknet.

Ausbeute: 3,6 g (77 % der Theorie)

Schmelzpunkt: 164-166 ° C

g) 1-Ethyl-7,8-difluor-1,4-dihydro-4-oxo-3-chinolincarbonsäure

3,5 g des unter f. erhaltenen Produktes werden in einem Gemisch aus 16 ml Essigsäure, 16 ml Wasser und 1,6 ml konzentrierter Schwefelsäure vier Stunden auf 140 ° C erwärmt. Der erkaltete Ansatz wird auf Eiswasser gegeben, das ausgefallene Produkt isoliert, mit Wasser nachgewaschen und bei 100 ° C getrocknet.

Ausbeute: 3,0 g (99 % der Theorie)

Schmelzpunkt: 237-239 ° C

Beispiel P

7,8-Difluor-1-(2,4-difluorphenyl)-1,4-dihydro-4-oxo-3-chinolincarbonsäure

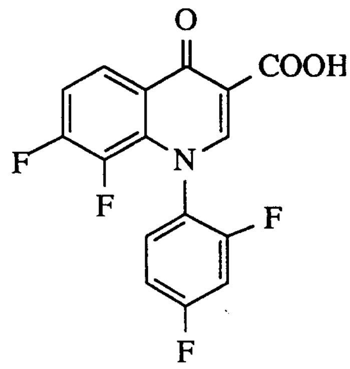

a) 2-(2,3,4-Trifluorbenzoyl)-3-(2,4-difluorphenylamino)-acrylsäureethylester
9,06 g des unter Od. erhaltenen Produktes werden mit 4,26 g (0,33 mol) 2,4-Difluoranilin analog Beispiel Oe. umgesetzt.
Ausbeute: 7,3 g (63 % der Theorie)
Schmelzpunkt: 123-125 ° C
b) 7,8-Difluor-1-(2,4-difluorphenyl)-1,4-dihydro-4-oxo-3-chinolincarbonsäureethylester
7,3 g (0,019 mol) des unter a. erhaltenen Produktes werden mit 2,96 g (0,021 mol) Kaliumcarbonat analog zum Beispiel Of. umgesetzt.
Ausbeute: 5,8 g (83 % der Theorie)
Schlmelzpunkt: 159-160 ° C
c) 7,8-Difluor-1-(2,4-difluorphenyl)-1,4-dihydro-4-oxo-3-chinolincarbonsäure
5,7 g (0,016 mol) des unter b. erhaltenen Produktes werden analog zum Beispiel Og. umgesetzt.
Ausbeute: 4,9 g (94 % der Theorie)
Schmelzpunkt: 222-224 ° C

Beispiel Q

8-Chlor-1-ethyl-7-fluor-1,4-dihydro-4-oxo-3-chinolincarbonsäure

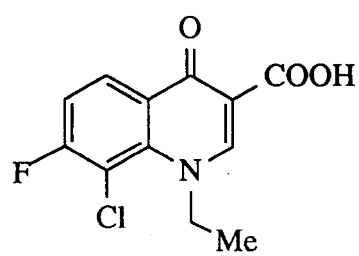

a) 2-(3-Chlor-2,4-difluorbenzoyl)-3-ethylamino-acrylsäureethylester
9,55 g (0,03 mol) 2-(3-Chlor-2,4-difluorbenzoyl)-3-ethoxyacrylsäureethylester werden in 66 ml Ethanol gelöst. Unter Eiskühlung werden 2,12 ml (0,033 mol) einer 70 %igen Ethylaminlösung zugetropft und unter Erwärmung auf Raumtemperatur über Nacht nachgerührt. Nach Zugabe von Wasser wird das Produkt isoliert, mit Wasser gewaschen und getrocknet.
Ausbeute: 6,3 g (66 % der Theorie)
Schmelzpunkt: 107-109 ° C
b) 8-Chlor-1-ethyl-7-fluor-1,4-dihydro-4-oxo-3-chinolin-carbonsäureethylester
3,175 g (0,01 mol) des unter a. erhaltenen Produktes werden mit 1,56 g (0,011 mol) Kaliumcarbonat in

20 ml Dimethylformamid vier Stunden auf 100 ° C erwärmt. Der erkaltete Ansatz wird auf Eiswasser gegeben, das Produkt wird isoliert, mit Wasser nachgewaschen und bei 100 ° C getrocknet.
Ausbeute: 2,8 g (94 % der Theorie)
Schmelzpunkt: 167-169 ° C
c) 8-Chlor-1-ethyl-7-fluor-1,4-dihydro-4-oxo-3-chinolincarbonsäure
2,7 g (9,1 mol) des unter b. erhaltenen Produktes werden in einer Mischung aus 11 ml Essigsäure, 11 ml Wasser und 1,1 ml konzentrierter Schwefelsäure vier Stunden zum Rückfluß erhitzt. Der Ansatz wird mit Wasser versetzt, das Produkt isoliert, mit Wasser nachgewaschen und bei 100 ° C getrocknet.
Ausbeute: 2,4g (98 % der Theorie)
Schmelzpunkt: 211-212 ° C

Beispiel R

8-Chlor-7-fluor-1-(2,4-difluorphenyl)-1,4-dihydro-4-oxo-3-chinolincarbonsäure

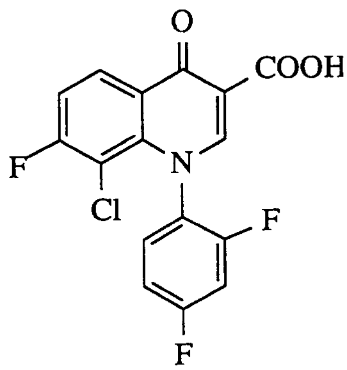

a) 2-(3-Chlor-1,4-difluorbenzoyl)-3-(2,4-difluorphenylamino)-acrylsäureethylester
Analog zum Beispiel Oa. werden 9,55 g (0,03 mol) 2-(3-Chlor-2,4-difluorbenzoyl)-3-ethoxyacrylsäureethylester und 4,26 g (0,033 mol) 2,4-Difluorphenylamin umgesetzt.
Ausbeute: 9,4g (78 % der Theorie)
Schmelzpunkt: 115-116 ° C
b) 8-Chlor-7-fluor-1-(2,4-difluorphenyl)-1,4-dihydro-4-oxo-3-chinolincarbonsäureethylester
4,01 g (0,01 mol) des bei a) erhaltenen Produktes werden analog zum Beispiel Ob. mit 1,56 g (0,011 mol) Kaliumcarbonat in Dimethylformamid umgesetzt.
Ausbeute: 3,3 g (86 % der Theorie)
Schmelzpunkt: 183-185 ° C
c) 8-Chlor-7-fluor-1-(2,4-difluorphenyl)-1,4-dihydro-4-oxo-3-chinolincarbonsäure
3,2 g (8,4 mmol) des bei b. erhaltenen Esters werden analog zum Beispiel Oc. verseift.
Ausbeute: 2,7 g (91 % der Theorie)
Schmelzpunkt: 204-206 ° C

Beispiel S

1-Cyclopropyl-7-fluor-1,4-dihydro-8-methyl-4-oxo-3-chinolincarbonsäure

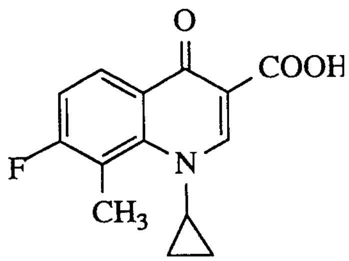

a) 3-Cyclopropylamino-2-(2,4-difluor-3-methylbenzoyl)-acrylsäureethylester
Zur bei 0 °C rührenden Lösung von 10 g (0,034 mol) 3-Ethoxy-2-(2,4-difluor-3-methylbenzoyl)-acrylsäureethylester (DE 3 615 767) werden 2,3 g (0,034 mol) Cyclopropylamin getropft und 30 Minuten bei Raumtemperatur nachgerührt. Der Ansatz wird auf Eiswasser gegeben und das ausgefallene Produkt mit Ethanol/Wasser gewaschen.
Ausbeute: 6,5 g (62 % der Theorie)
Schmelzpunkt: 96-97 °C
b) 1-Cyclopropyl-7-fluor-1,4-dihydro-8-methyl-4-oxo-3-chinolincarbonsäureethylester
6,5 g (0,021 mol) des unter a. erhaltenen Produktes werden mit 3,4 g (0,025 mol) Kaliumcarbonat in 30 ml Dimethylformamid auf 140 °C erwärmt. Der erkaltete Ansatz wird auf Eiswasser gegeben, das Produkt isoliert, mit Wasser gewaschen und bei 100 °C getrocknet.
Ausbeute: 5,5g (90 % der Theorie)
Schmelzpunkt: 167-168 °C
c) 1-Cyclopropyl-7-fluor-1,4-dihydro-8-methyl-4-oxo-3-chinolincarbonsäure
4,5 g (0,016 mol) des unter b. erhaltenen Esters werden in einer Mischung aus 18 ml Essigsäure, 14 ml Wasser und 1,6 ml konzentrierter Schwefelsäure zwei Stunden zum Rückfluß erhitzt. Der Ansatz wird auf Wasser gegeben, das Produkt isoliert und gründlich mit Wasser gewaschen.
Ausbeute: 3,9 g (93 % der Theorie)
Schmelzpunkt: 236-238 °C

Beispiel T

7-Chlor-1-cyclopropyl-1,4-dihydro-4-oxo-1,8-naphthyndin-3-carbonsäureethylester

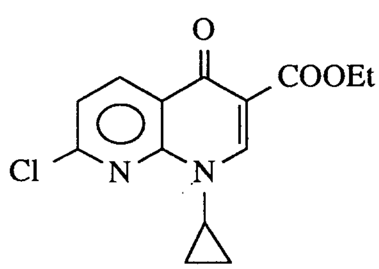

a) (2,6-Dichlornicotinoyl)malonsäurediethylester
Man legt 7,21 g (0,075 mol) Magnesiumchlorid bei 0 °C in 75 ml absolutem Acetonitril vor und tropft unter Eisbadkühlung 12,12 g (0,075 mol) Malonsäurediethylester zu. Anschließend werden 15,34 g (0,150 mol) Triethylamin bei 0 °C zugetropft, nach 60-minütigem Nachrühren werden bei 0 °C 17,0 g (0,075 mol) 2,6-Dichlornicotinsäurechlorid (Helvetia Chimica Acta 59, 222, (1976)) zugetropft und unter Erwärmung auf Raumtemperatur über Nacht nachgerührt. Es wird mit 80 ml 18 %iger Salzsäure versetzt und mit Methyl-tert.-butylether extrahiert, über Natriumsulfat getrocknet und im Vakuum eingeengt.

b) (2,6-Dichlornicotinoyl)essigsäureethylester

Der rohe (2,6-Dichlornicotinoyl)-malonsäurediethylester wird in 45 ml Wasser mit 90 mg p-Toluolsulfonsäure 2 Stunden zum Rückfluß erhitzt. Es wird mit Methylenchlorid extrahiert, mit Wasser gewaschen, über Natriumsulfat getrocknet und im Vakuum eingeengt. Das Rohprodukt wird an Kieselgel (Laufmittel Dichlormethan) gereinigt.

Ausbeute: 14,2 g (72 % der Theorie über zwei Schritte)

c) 2-(2,6-Dichlornicotinoyl)-3-ethoxyacrylsäureethylester

43 g (0,162 mol) des Produktes von b. werden in 38,1 g (0,26 mol) Orthoameisensäureethylester und 42,4 g (0,42 mol) Essigsäureanhydrid zwei Stunden auf 150-160 °C erwärmt. Alle leicht flüchtigen Bestandteile werden im Hochvakuum bei einer Badtemperatur bis 100 °C abdestilliert und das Rohprodukt direkt weiter umgesetzt.

Rohausbeute: 50,5 g

d) 3-Cyclopropylamino-2-(2,6-dichlornicotinoyl)-acrylsäureethylester

Zu 9,54 g des Rohproduktes von c. werden in 66 ml Ethanol bei 0 °C 1,88 g (0,33 mol) Cyclopropylamin getropft und über Nacht bei Raumtemperatur nachgerührt. Es wird mit Wasser versetzt und das auskristallisierte Produkt isoliert.

Ausbeute: 9,3 g (94 % der Theorie)

Schmelzpunkt: 123-124 °C

e) 1-Cyclopropyl-7-chlor-1,4-dihydro-4-oxo-1,8-naphthyridin-3-carbonsäureethylester

3,29 g (0,01 mol) des unter d. erhaltenen Produktes werden in 20 ml Dimethylformamid mit 1,6 g (0,019 mol) Kaliumcarbonat eine Stunde auf 100 °C erwärmt. Nach dem Abkühlen wird mit Wasser versetzt, das auskristallisierte Produkt isoliert und bei 100 °C getrocknet.

Ausbeute: 2,8 g (95 % der Theorie)

Schmelzpunkt: 187-190 °C

Beispiel U

7-Chlor-1-(2,4-difluorphenyl)-1,4-dihydro-4-oxo-1,8-naphthyridin-3-carbonsäureethylester

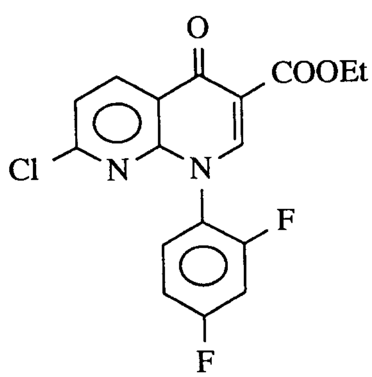

a) 2-(2,6-Dichlornicotinoyl)-3-(2,4-difluorphenylamino)-acrylsäureethylester

Zu 9,54 g des Rohproduktes von Tc. werden in 66 ml Ethanol bei 0 °C 4,26 g (0,33 mol) 2,4-Difluorphenylamin getropft und über Nacht bei Raumtemperatur nachgerührt. Es wird mit Wasser versetzt und das auskristallisierte Produkt isoliert.

Ausbeute: 8,8 g (73 % der Theorie)

Schmelzpunkt: 128-129 °C

b) 7-Chlor-1-(2,4-difluorphenyl)-1,4-dihydro-4-oxo-1,8-naphthyridin-3-carbonsäureethylester

4,01 g (0,01 mol) des unter a. erhaltenen Produktes werden in 20 ml Dimethylformamid mit 1,6 g (0,019 mol) Kaliumcarbonat eine Stunde auf 100 °C erwärmt. Nach dem Abkühlen wird mit Wasser versetzt und das auskristallisierte Produkt isoliert und bei 100 °C getrocknet.

Ausbeute: 3,5 g (96 % der Theorie)

Schmelzpunkt: 200-202 °C

Beispiel V

8-Chlor-7-fluor-1-(cis-2-fluorcyclopropyl)-1,4-dihydro-4-oxo-3-chinolincarbonsäure

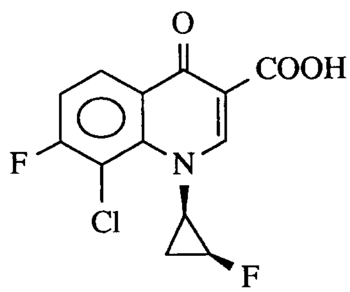

a) 2-(3-Chlor-2,4-difluorbenzoyl)-3-(cis-2-fluorcyclopropylamino)-acrylsäureethylester
4,8 g (0,015 mol) 2-(3-Chlor-2,4-difluorbenzoyl)-3-ethoxyacrylsäureethylester und 1,67 g (0,015 mol) cis-2-Fluorcyclopropylaminhydrochlorid (JP 3291258 A2) werden bei 0 °C in einer Mischung aus 22,5 ml Dichlormethan und 9 ml Wasser vorgelegt. Bei 0 °C wird anschließend die Lösung von 1,275 g Natriumhydrogencarbonat in 15 ml Wasser zugetropft und unter Erwärmung auf Raumtemperatur über Nacht nachgerührt. Die Phasen werden getrennt, mit Dichlormethan reextrahiert, über Natriumsulfat getrocknet und das Lösungmittel im Vakuum entfernt.
Ausbeute: 4,9 g (94 % der Theorie), Öl
b) 8-Chlor-7-fluor-1-(cis-2-fluorcyclopropyl)-1,4-dihydro-4-oxo-3-chinolincarbonsäureethylester
4,0 g (0,012 mol) des unter a. erhaltenen Produktes werden mit 1,9 g (0,014 mol) Kaliumcarbonat in 25 ml Dimethylformarmid vier Stunden auf 100 °C erwärmt. Der erkaltete Ansatz wird auf Eiswasser gegeben und mit Dichlormethan extrahiert Die organischen Phasen werden über Natriumsulfat getrocknet, das Lösungsmittel im Vakuum entfernt, das Rohprodukt wird mit Acetonitril verrührt und das Produkt isoliert.
Ausbeute: 1,9 g (48 % der Theorie)
Schmelzpunkt: 179-180 °C
c) 8-Chlor-7-fluor-1-(cis-2-fluorcyclopropyl)-1,4-dihydro-4-oxo-3-chinolincarbonsäure
1,0 g (3 mmol) des unter b. erhaltenen Produktes werden in einer Mischung aus 4 ml Essigsäure, 4 ml Wasser und 0,4 ml konzentrierter Schwefelsäure vier Stunden zum Rückfluß erhitzt. Der Ansatz wird mit Eiswassser versetzt, das Produkt isoliert, mit Wasser nachgewaschen und bei 100 °C getrocknet.
Ausbeute: 0,76 g (85 % der Theorie)
Schmelzpunkt: 178-180 °C

Beispiel W

8-Chlor-7-fluor-1-(trans-2-fluorcyclopropyl)-1,4-dihydro-4-oxo-3-chinolincarbonsäure

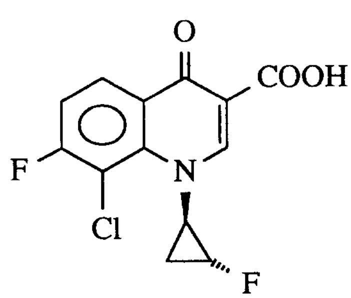

a) 2-(3-Chlor-2,4-difluorbenzoyl)-3-(trans-2-fluorcyclopropylamino)-acrylsäureethylester
4,8 g (0,015 mol) 2-(3-Chlor-2,4-difluorbenzoyl)-3-ethoxyacrylsäureethylester und 1,67 g (0,015 mol) trans-2-Fluorcyclopropylaminhydrochlorid (JP 3291258 A2) werden bei 0 °C in einer Mischung aus 22,5

ml Dichlormethan und 9 ml Wasser vorgelegt. Bei 0 °C wird anschließend die Lösung von 1,275 g Natriumhydrogencarbonat in 15 ml Wasser zugetropft und unter Erwärmung auf Raumtemperatur über Nacht nachgerührt. Die Phasen werden getrennt, mit Dichlormethan reextrahiert, über Natriumsulfat getrocknet und das Lösungsmittel im Vakuum entfernt.
Ausbeute: 5,0 g (96 % der Theorie), Öl
b) 8-Chlor-7-fluor-1-(trans-2-fluorcyclopropyl)-1,4-dihydro-4-oxo-3-chinolincarbonsäureethylester
4,2 g (0,012 mol) des unter a. erhaltenen Produktes werden mit 1,9 g (0,014 mol) Kaliumcarbonat in 25 ml Dimethylformamid vier Stunden auf 100 °C erwärmt. Der erkaltete Ansatz wird auf Eiswasser gegeben und das Produkt isoliert.
Ausbeute: 3,0 g (76 % der Theorie)
Schmelzpunkt: 184-186 °C
c) 8-Chlor-7-fluor-1-(trans-2-fluorcyclopropyl)-1,4-dihydro-4-oxo-3-chinolincarbonsäure
2,3 g (7 mmol) des unter b. erhaltenen Produktes werden in einer Mischung aus 9,2 ml Essigsäure, 9,2 ml Wasser und 0,9 ml konzentrierter Schwefelsäure vier Stunden zum Rückfluß erhitzt. Der Ansatz wird mit Eiswasser versetzt, das Produkt isoliert, mit Wasser nachgewaschen und bei 100 °C getrocknet.
Ausbeute: 1,8 g (86 % der Theorie)
Schmelzpunkt: 234-236 °C

Beispiel X

7,8-Difluor-1-(cis-2-fluorcyclopropyl)-1,4-dihydro-4-oxo-3-chinolincarbonsäure

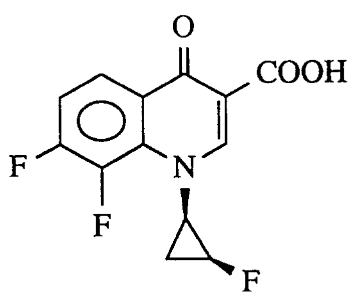

a) 2-(2,3,4-Trifluorbenzoyl)-3-(cis-2-fluorcyclopropylamino)acrylsäureethylester
4,53 g (0,015 mol) 2-(2,3,4-Trifluorbenzoyl)-3-ethoxy-acrylsäureethylesterund 1,67 g (0,015 mol) cis-2-Fluorcyclopropylaminhydrochlorid (JP 3291258 A2) werden bei 0 °C in einer Mischung aus 22,5 ml Dichlormethan und 9 ml Wasser vorgelegt. Bei 0 °C wird anschließend die Lösung von 1,275 g Natriumhydrogencarbonat in 15 ml Wasser zugetropft und unter Erwärmung auf Raumtemperatur über Nacht nachgerührt. Die Phasen werden getrennt, mit Dichlormethan reextrahiert, über Natriumsulfat getrocknet und das Lösungsmittel im Vakuum entfernt.
Ausbeute: 4,7 g (94 % der Theorie)
Schmelzpunkt: 78-80 °C
b) 7,8-Difluor-1-(cis-2-fluorcyclopropyl)-1,4-dihydro-4-oxo-3-chinolincarbonsäureethylester
4,5 g (0,0136 mol) des unter a. erhaltenen Produktes werden mit 2,12 g (0,015 mol) Kaliumcarbonat in 25 ml Dimethylformamid zwei Stunden auf 100 °C erwärmt. Der erkaltete Ansatz wird auf Eiswasser gegeben und das Produkt isoliert.
Ausbeute: 3,1 g (74 % der Theorie)
Schmelzpunkt: 189-190 °C
c) 7,8-Difluor-1-(cis-2-fluorcyclopropyl)-1,4-dihydro-4-oxo-3-chinolincarbonsäure
3,1 g (10 mmol) des unter b. erhaltenen Produktes werden in einer Mischung aus 13 ml Essigsäure, 13 ml Wasser und 1,3 ml konzentrierter Schwefelsäure zwei Stunden zum Rückfluß erhitzt. Der Ansatz wird mit Eiswasser versetzt, das Produkt isoliert, mit Wasser nachgewaschen und bei 100 °C getrocknet.
Ausbeute: 2,4 g (85 % der Theorie)
Schmelzpunkt: 229-230 °C

Beispiel Y

1-Cyclopropyl-7-fluor-8-trifluormethyl-1,4-dihydro-4-oxo- 3-chinolincarbonsäure

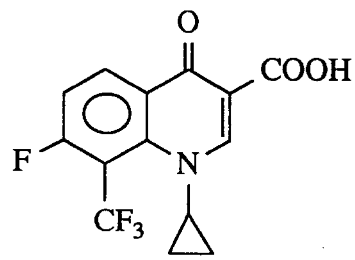

a) 2,4-Difluor-3-trifluormethylbenzoesäure

Unter Stickstoff werden 91 g (0,5 mol) 1,3-Difluor-2-trifluormethylbenzol in einem Gemisch aus 300 ml absolutem Tetrahydrofuran und 300 ml absolutem Diethylether gelöst und bei -70 °C tropfenweise mit 220 ml (0,55 mol) einer 2,5 M n-Butyllithium-Lösung in Hexan versetzt. Nach einstündigem Nachrühren werden ca. 200 g Kohlendioxid bei -20 °C durch die Lösung geleitet. Anschließend läßt man auf 5 °C auftauen, rührt eine Stunde nach und hydrolysiert mit 200 ml 2 N Salzsäure. Die wäßrige Phase wird abgetrennt und zweimal mit Diethylether extrahiert Die vereinigten organischen Phasen werden mit verdünnter Salzsäure und Wasser gewaschen, über Magnesiumsulfat getrocknet und einrotiert.

Ausbeute: 48,6 g (43 % der Theorie)

Schmelzpunkt: 70-71 °C

b) 2,4-Difluor-3-trifluormethylbenzoesäurechlorid

Zu 130 ml Thionylchlorid werden bei Raumtemperatur portionsweise 19,3 g (0,085 mol) 2,4-Difluor-3-trifluormethylbenzoesäure eingetragen. Nach dem Ende der Dosierung wird so lange auf 50 °C erwärmt bis die Gasentwicklung beendet ist. Danach wird das überschüssige Thionylchlorid durch Destillation abgetrennt und das Rohprodukt direkt weiter umgesetzt.

Ausbeute: 20,0 g (90 % der Theorie)

c) (2,4-Difluor-3-trifluormethylbenzoyl)malonsäurediethylester

2,15 g (0,09 mol) Magnesiumspäne werden in 4,8 ml Ethanol vorgelegt, die Reaktion mit einigen Tropfen Tetrachlorkohlenstoff gestartet und anschließend die Lösung von 12,8 g (0,075 mol) Malonsäurediethylester in 9 ml Ethanol und 35 ml Toluol so zugetropft, daß die Innentemperatur zwischen 50 und 60 °C liegt. Anschließend wird eine Stunde bei 60 °C nachgerührt. Bei -10 bis -5 °C wird die Lösung von 20,0 g (0,082 mol) 2,4-Difluor-3-trifluormethylbenzoesäurechlorid in 9 ml Toluol zugetropft, eine Stunde bei 0 °C und anschließend unter Erwärmung auf Raumtemperatur nachgerührt. Der Ansatz wird auf 35 ml Eiswasser gegeben mit 5,7 ml konzentrierter Schwefelsäure versetzt und mit Toluol extrahiert. Es wird mit gesättigter Natriumchloridlösung gewaschen und das Lösungsmittel im Vakuum entfernt.

Rohausbeute: 30,0 g

d) (2,4-Difluor-3-trifluormethylbenzoyl)essigsäureethylester

30,0 g des bei c. erhaltenen Rohproduktes werden in 30 ml Wasser mit 0,96 g p-Toluolsulfonsäure 4,5 Stunden zum Rückfluß erhitzt. Der erkaltete Ansatz wird mit Methylenchlorid extrahiert, mit gesättigter Natriumchloridlösung gewaschen, über Natriumsulfat getrocknet und im Vakuum eingeengt.

Rohausbeute: 22 g

e) 3-Ethoxy-2-(2,4-difluor-3-trifluormethylbenzoyl)-acrylsäureethylester

22 g des unter d. erhaltenen Produktes werden mit 17,4 g (0,12 mol) Orthoameisensäureethylester und 19,4 g (0,19 mol) Essigsäureanhydrid zwei Stunden auf 150-160 °C erwärmt. Überschüssiges Reagenz wird zunächst im Vakuum, dann im Hochvakuum bis zu einer Badtemperatur von 100 °C entfernt.

Rohausbeute: 23,1 g

f) 3-Cyclopropylamino-2-(2,4-difluor-4-trifluormethylbenzoyl)-acrylsäureethylester

23,0 g (0,065 mol) des unter e. erhaltenen Produktes werden in 140 ml Ethanol bei 0 °C vorgelegt und 4,08 g (0,072 mol) Cyclopropylamin zugetropft. Es wird über Nacht bei Raumtemperatur nachgerührt, 140 ml Wasser zugetropft und das ausfallende Produkt isoliert. Es wird mit Wasser nachgewaschen und bei ca. 100 °C getrocknet.

Ausbeute: 9,4 g (39 % der Theorie)

Schmelzpunkt: 104-105 °C

g) 1-Cyclopropyl-7-fluor-8-trifluormethyl-1,4-dihydro-4-oxo-3-chinolincarbonsäureethylester

9,0 g (0,025 mol) des unter f. erhaltenen Produktes werden mit 3,9 g (0,028 mol) Kaliumcarbonat in 50 ml Dimethylformamid vier Stunden auf 100 °C erwärmt. Der erkaltete Ansatz wird auf Eiswasser gegeben, das Produkt isoliert, mit Wasser nachgewaschen und bei 100 °C getrocknet.

Ausbeute: 8,1 g (98 % der Theorie)

Schmelzpunkt: 154-155 °C

h) 1-Cyclopropyl-7-fluor-8-trifluormethyl-1,4-dihydro-4-oxo-3-chinolincarbonsäure

8,0 g (0,023 mol) des unter g. erhaltenen Produktes werden in einem Gemisch aus 30 ml Essigsäure, 30 ml Wasser und 3 ml konzentrierter Schwefelsäure zwei Stunden auf 140 °C erwärmt. Der erkaltete Ansatz wird auf Eiswasser gegeben, das ausgefallene Produkt isoliert, mit Wasser nachgewaschen und bei 100 °C getrocknet.

Ausbeute: 7,0 g (96 % der Theorie)

Schmelzpunkt: 209-210 °C

Beispiel Z

1-Cyclopropyl-5,7-difluor-8-trifluormethyl-1,4-dihydro-4-oxo-3-chinolincarbonsäure

a) 2,4,6-Trifluor-3-trifluormethylbenzoesäure

Analog zum Beispiel Ya. wird bei der Umsetzung von 1,3,5-Trifluor-2-trifluormethylbenzol die Titelverbindung erhalten.

Schmelzpunkt: 70-71 °C

b) 2,4,6-Trifluor-3-trifluormethylbenzoesäurechlorid

Zu 40 ml Thionylchlorid werden bei Raumtemperatur portionsweise 5,8 g (0,024 mol) 2,4,6-Trifluor-3-trifluormethylbenzoesäure gegeben. Nach dem Ende der Dosierung wird so lange auf 50 °C erwärmt bis die Gasentwicklung beendet ist. Danach wird das überschüssige Thionylchlorid durch Destillation abgetrennt und das Rohprodukt direkt weiter umgesetzt.

Ausbeute: 6,0 g (95 % der Theorie)

c) (2,4,6-Trifluor-3-trifluormethylbenzoyl)malonsäurediethylester

0,58 g (0,024 mol) Magnesiumspäne werden in 1,3 ml Ethanol vorgelegt, die Reaktion mit einigen Tropfen Tetrachlorkohlenstoff gestartet und anschließend die Lösung von 3,4 g (0,019 mol) Malonsäurediethylester in 2,4 ml Ethanol und 9 ml Toluol so zugetropft, daß die Innentemperatur zwischen 50 und 60 °C liegt. Anschließend wird eine Stunde bei 60 °C nachgerührt. Bei -10 bis -5 °C wird die Lösung von 5,8 g (0,027 mol) 2,4,6-Trifluor-3-trifluormethylbenzoesäurechlorid in 2,5 ml Toluol zugetropft, eine Stunde bei 0 °C und anschließend unter Erwärmung auf Raumtemperatur nachgerührt. Der Ansatz wird auf 10 ml Eiswasser gegeben, mit 1,5 ml konzentrierter Schwefelsäure versetzt und mit Toluol extrahiert. Es wird mit gesättigter Natriumchloridlösung gewaschen und das Lösungsmittel im Vakuum entfernt.

Rohausbeute: 8,6 g

d) (2,4,6-Trifluor-3 trifluormethylbenzoyl)essigsäureethylester

8,6 g des bei c. erhaltenen Rohproduktes werden in 9 ml Wasser mit 0,26 g p-Toluolsulfonsäure 4,5 Stunden zum Rückfluß erhitzt. Der erkaltete Ansatz wird mit Methylenchlorid extrahiert, mit gesättigter Natriumchloridlösung gewaschen, über Natriumsulfat getrocknet und im Vakuum eingeengt.

Rohausbeute: 5,6 g

e) 3-Ethoxy-2-(2,4,6-trifluor-3-trifluormethylbenzoyl)-acrylsäureethylester

5,4 g (0,017 mol) des unter d. erhaltenen Produktes werden mit 4,0 g (0,027 mol) Orthoameisensäureethylester und 4,45 g (0,043 mol) Essigsäureanhydrid zwei Stunden auf 150-160 °C erwärmt. Überschüssiges Reagenz wird zunächst im Vakuum, dann im Hochvakuum bis zu einer Badtemperatur von 100 °C entfernt.

Rohausbeute: 3,8 g

f) 3-Cyclopropylamino-2-(2,4,6-trifluor-4-trifluormethylbenzoyl)-acrylsäureethylester

3,8 g (0,01 mol) des unter e. erhaltenen Produktes werden in 22 ml Ethanol bei 0 °C vorgelegt und 0,63 g (0,011 mol) Cyclopropylamin zugetropft. Es wird über Nacht bei Raumtemperatur nachgerührt, 22 ml Wasser zugetropft und das ausfallende Produkt isoliert. Es wird mit Wasser nachgewaschen und bei ca. 100 °C getrocknet.

Ausbeute: 3,3 g (86 % der Theorie)

Schmelzpunkt: 146-148 °C

g) 1-Cyclopropyl-5,7-difluor-8-trifluormethyl-1,4-dihydro-4-oxo-3-chinolincarbonsäureethylester

3,5 g (9,2 mmol) des unter f. erhaltenen Produktes werden mit 1,45 g (0,01 mol) Kaliumcarbonat in 18 ml

Dimethylformamid eine Stunde auf 100 °C erwärmt. Der erkaltete Ansatz wird auf Eiswasser gegeben, das Produkt isoliert, mit Wasser nachgewaschen. Die Reinigung des Produktes erfolgt an Kieselgel, Laufmittel Cyclohexan/Tetrahydrofuran 7/3
Ausbeute: 1,4 g (42 % der Theorie)
Schmelzpunkt: 197-198 °C
h) 1-Cyclopropyl-5,7-difluor-8-trifluormethyl-1,4-dihydro-4-oxo-3-chinolincarbonsäure
1,4 g (3,9 mmol) des unter g. erhaltenen Produktes werden in einem Gemisch aus 5 ml Essigsäure, 5 ml Wasser und 0,5 ml konzentrierter Schwefelsäure zwei Stunden auf 140 °C erwärmt. Der erkaltete Ansatz wird auf Eiswasser gegeben, das ausgefallene Produkt isoliert, mit Wasser nachgewaschen und bei 100 °C getrocknet.
Ausbeute: 1,1 g (84 % der Theorie)
Schmelzpunkt: 208-210 °C

Beispiel AA

1-Cyclopropyl-7,8-difluor-5-trifluormethyl-1,4-dihydro-4-oxo-3-chinolincarbonsäure

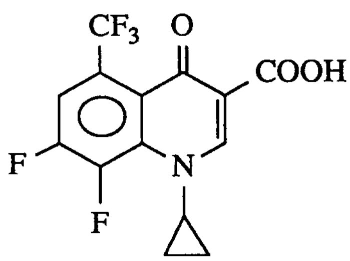

a) 2,3,4-Trifluor-6-trifluormethylbenzoesäure
Analog zum Beispiel Ya. wird bei der Umsetzung von 1,2,3,-Trifluor-5-trifluormethylbenzol die Titelverbindung erhalten.
Schmelzpunkt: 83-84 °C
b) 2,3,4-Trifluor-6-trifluormethylbenzoesäurechlorid
Zu 40 ml Thionylchlorid werden bei Raumtemperatur portionsweise 5,8 g (0,024 mol) 2,3,4,-Trifluor-6-trifluormethylbenzoesäure eingetragen. Nach dem Ende der Dosierung wird so lange auf 50 °C erwärmt bis die Gasentwicklung beendet ist. Danach wird das überschüssige Thionylchlorid durch Destillation abgetrennt und das Rohprodukt direkt weiter umgesetzt.
Ausbeute: 5,0 g (79 % der Theorie)
c) (2,3,4-Trifluor-6-trifluormethylbenzoyl)malonsäurediethylester
0,47 g (0,019 mol) Magnesiumspäne werden in 1,0 ml Ethanol vorgelegt, die Reaktion mit einigen Tropfen Tetrachlorkohlenstoff gestartet und anschließend die Lösung von 2,8 g (0,016 mol) Malonsäurediethylester in 2,0 ml Ethanol und 7,5 ml Toluol so zugetropft, daß die Innentemperatur zwischen 50 und 60 °C liegt. Anschließend wird eine Stunde bei 60 °C nachgerührt. Bei -10 bis -5 °C wird die Lösung von 4,8 g (0,027 mol) 2,3,4-Trifluor-6-trifluormethylbenzoesäurechlorid in 2,0 ml Toluol zugetropft, eine Stunde bei 0 °C und anschließend unter Erwärmung auf Raumtemperatur nachgerührt. Der Ansatz wird auf 10 ml Eiswasser gegeben, mit 1,25 ml konzentrierter Schwefelsäure versetzt und mit Toluol extrahiert. Es wird mit gesättigter Natriumchloridlösung gewaschen und das Lösungsmittel im Vakuum entfernt.
Rohausbeute: 6,6 g
d) (2,3,4-Trifluor-6-trifluormethylbenzoyl)essigsäureethylester
6,6 g des bei c. erhaltenen Rohproduktes werden in 7,5 ml Wasser mit 0,21 g p-Toluolsulfonsäure 4,5 Stunden zum Rückfluß erhitzt. Der erkaltete Ansatz wird mit Methylenchlorid extrahiert, mit gesättigter Natriumchloridlösung gewaschen, über Natriumsulfat getrocknet und im Vakuum eingeengt.
Rohausbeute: 4,2 g
e) 3-Ethoxy-2-(2,3,4-trifluor-6-trifluormethylbenzoyl)-acrylsäureethylester
4,0 g (0,013 mol) des unter d. erhaltenen Produktes werden mit 3,5 g (0,024 mol) Orthoameisensäureethylester und 3,4 g (0,033 mol) Essigsäureanhydrid zwei Stunden auf 150-160 °C erwärmt. Überschüssiges

Reagenz wird zunächst im Vakuum, dann im Hochvakuum bis zu einer Badtemperatur von 100 ° C entfernt.
Rohausbeute: 2,7 g
f) 3-Cyclopropylamino-2-(2,3,4-trifluor-6-trifluormethylbenzoyl)-acrylsäureethylester
2,7 g (7,3 mmol) des unter e. erhaltenen Produktes werden in 16 ml Ethanol bei 0 ° C vorgelegt und 0,46 g (8 mmol) Cyclopropylamin zugetropft. Es wird über Nacht bei Raumtemperatur nachgerührt, 16 ml Wasser zugetropft und das ausfallende Produkt isoliert. Es wird mit Wasser nachgewaschen und bei ca. 100 ° C getrocknet.
Ausbeute: 2,1 g (75 % der Theorie)
Schmelzpunkt: 165-168 ° C
g) 1-Cyclopropyl-7,8-difluor-5-trifluormethyl-1,4-dihydro-4-oxo-3-chinolincarbonsäureethylester
2,1 g (5,5 mmol) des unter f. erhaltenen Produktes werden mit 0,88 g (6,4 mmol) Kaliumcarbonat in 11 ml Dimethylformamid eine Stunde auf 100 ° C erwärmt. Der erkaltete Ansatz wird auf Eiswasser gegeben, das Produkt isoliert, mit Wasser nachgewaschen.
Ausbeute: 1,7 g (85 % der Theorie)
Schmelzpunkt: 188-190 ° C
h) 1-Cyclopropyl-7,8-difluor-5-trifluormethyl-1,4-dihydro-4-oxo-3-chinolincarbonsäure
1,5 g (4,2 mmol) des unter g. erhaltenen Produktes werden in einem Gemisch aus 5,5 ml Essigsäure, 5,5 ml Wasser und 0,55 ml konzentrierter Schwefelsäure zwei Stunden auf 140 ° C erwärmt. Der erkaltete Ansatz wird auf Eiswasser gegeben, das ausgefallene Produkt isoliert, mit Wasser nachgewaschen und bei 100 ° C getrocknet.
Ausbeute: 1,3 g (92 % der Theorie)
Schmelzpunkt: 226-228 ° C

## Herstellung der Wirkstoffe

### Beispiel 1

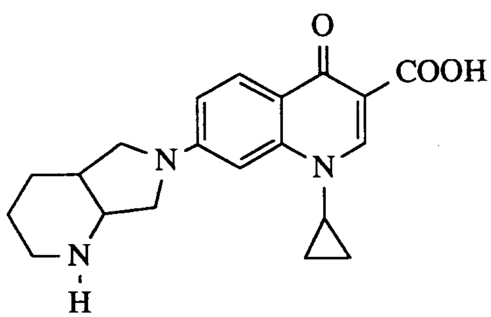

7-(2,8-Diazabicyclo[4.3.0]nonan-8-yl)-1-cyclopropyl-1,4-dihydro-4-oxo-3-chinolincarbonsäure

741 mg (3 mmol) 1-Cyclopropyl-7-fluor-1,4-dihydro-4-oxo-3-chinolincarbonsäure werden mit 567 mg (4,5 mol) 2,8-Diazabicyclo[4.3.0]nonan und 672 mg 1,4-Diazabicyclo[2.2.2]octan in 30 ml N-Methylpyrrolidon zwei Stunden auf 100 ° C erwärmt. Die Mischung wird im Hochvakuum eingeengt, der Rückstand mit Acetonitril gründlich verrührt und bei 100 ° C getrocknet.
Ausbeute: 820 mg (77 % der Theorie)
Schmelzpunkt: 250-252 ° C (unter Zersetzung)

Beispiel 2

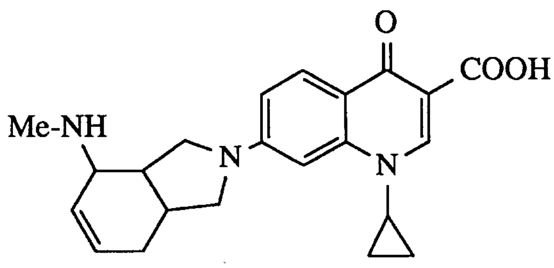

1-Cyclopropyl-1,4-dihydro-7-(4-methylamino-1,3,3a,4,7,7a-hexahydroisoindol-2-yl)-4-oxo-3-chinolincarbonsäure

Analog zum Beispiel 1 wird bei der Umsetzung mit 4-Methylamino-1,3,3a,4,7,7a-hexahydroisoindol die Titelverbindung erhalten.
Schmelzpunkt: 238-240 °C

Beispiel 3

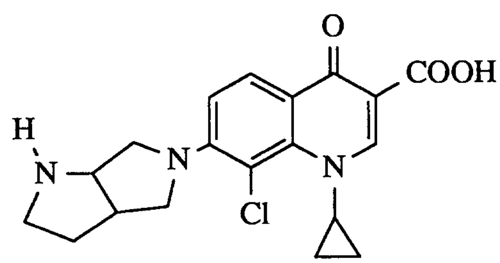

7-(2,7-Diazabicyclo[3.3.0]octan-7-yl)-8-chlor-1-cyclopropyl-1,4-dihydro-4-oxo-3-chinolincarbonsäure

650 mg (2,3 mmol) 8-Chlor-1-cyclopropyl-7-fluor-1,4-dihydro-4-oxo-3-chinolincarbonsäure werden mit 315 mg (2,8 mmol) 2,7-Diazabicyclo[3.3.0]octan und 510 mg (4,6 mmol) 1,4-Diazabicyclo[2.2.2]octan in 23 ml Dimethylsulfoxid sechs Stunden auf 120 °C erwärmt. Alle flüchtigen Komponenten werden im Hochvakuum entfernt, der Rückstand gründlich mit Acetonitril verrührt und bei ca 100 °C getrocknet.
Ausbeute: 629 mg (72 % der Theorie)
Schmelzpunkt: 202-204 °C (unter Zersetzung)

Analog Beispiel 3 wurden hergestellt:

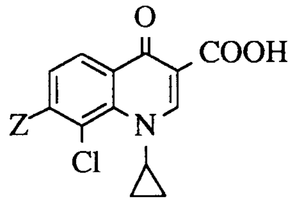

| Beispiel | Z | Schmelzpunkt [°C] |
|---|---|---|
| 4 | 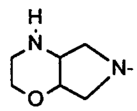 | 250-255 |
| 5 | 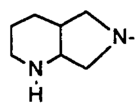 | 255-259 |
| 6 | 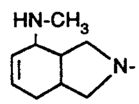 | 213-217 |
| 7 | 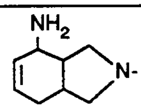 | 184-186 (Zers.) |
| 8 | 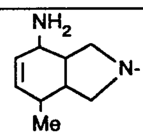 | 169-171 (Zers.) |
| 9 | 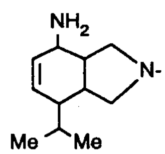 | 145-147 (Zers.) |
| 10 | 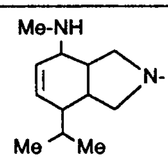 | 170-180 (Zers.) aus Acetonitril |
| 11 | 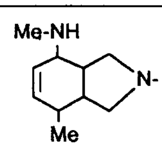 | 168-170 (Zers.) |

Fortsetzung

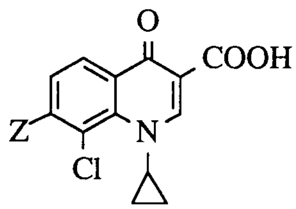

| Beispiel | Z | Schmelzpunkt [°C] |
|---|---|---|
| 12 | 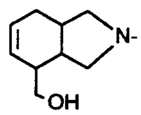 | 214-216 (Zers.) |
| 13 | 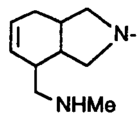 | 244-246 (Zers.) |
| 14 | 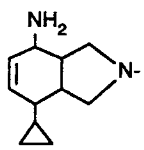 | 184-186 (Zers.) |

Beispiel 15

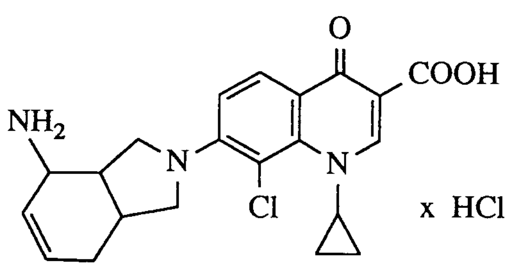

x HCl

7-(4-Amino-1,3,3a,4,7,7a,hexahydroisoindol-2-yl)-8-chlor-1-cyclopropyl-1,4-dihydro-4-oxo-3-chinolin carbonsäure hydrochlorid

2 g (5 mmol) 7-(4-Amino-1,3,3a,4,7,7a-hexahydroisoindol-2-yl)-3-chlor-1-cyclopropyl-1,4-dihydro-4-oxo-3-chinolincarbonsäure werden in 60 ml halbkonzentrierter Salzsäure 30 Minuten bei 60 °C gerührt. Überschüssige Salzsäure wird im Vakuum entfernt und der Rückstand mit Acetonitril verrührt.
Ausbeute: 1,8 g (82 % der Theorie)
Schmelzpunkt: 118-120 °C (unter Zersetzung)

Beispiel 16

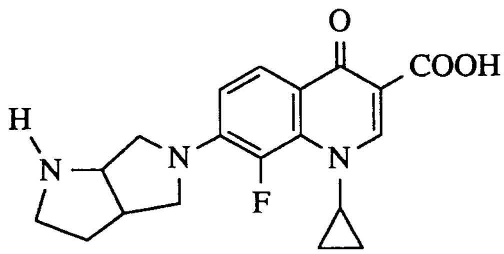

7-(2,7-Diazabicyclo[3.3.0]octan-7-yl)-1-cyclopropyl-8-fluor-1,4-dihydro-4-oxo-3-chinolincarbonsäure

620 mg (2,3 mmol) 1-Cyclopropyl-7,8-difluor-1,3-dihydro-4-oxo-3-chinolincarbonsäure werden mit 315 mg (2,8 mmol) 2,7-Diazabicyclo[3.3.0]octan und 0,51 g (4,6 mmol) 1,4-Diazabicyclo[2.2.2]octan in 23 ml Dimethylsulfoxid zwei Stunden auf 120 ° C erwärmt. Alle flüchtigen Bestandteile werden im Hochvakuum entfernt und der Rückstand gründlich mit Acetonitril verrührt und bei ca. 100 ° C getrocknet.
Ausbeute: 770 mg (93 % der Theorie)
Schmelzpunkt: 249-251 ° C (unter Zersetzung)

Analog Beispiel 16 wurden hergestellt:

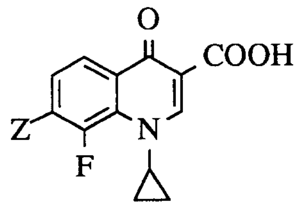

| Beispiel | Z | Schmelzpunkt [°C] |
|---|---|---|
| 17 | 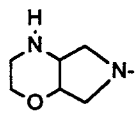 | 224-228 |
| 18 | 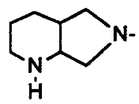 | 212-216 |
| 19 | 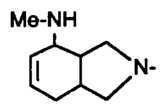 | 204-210 |
| 20 | 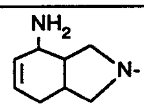 | 158-160 (Zers.) |
| 21 | 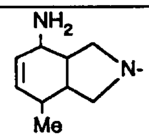 | 200-204 (Zers.) |
| 22 | 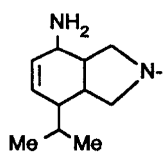 | 250-252 (Zers.) |
| 23 | 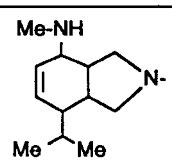 | 228-230 (Zers.) |
| 24 | 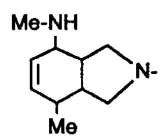 | 191-193 (Zers.) |

Fortsetzung

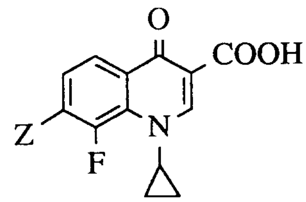

| Beispiel | Z | Schmelzpunkt [°C] |
|---|---|---|
| 25 | 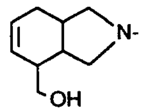 | 248-250 (Zers.) |
| 26 | 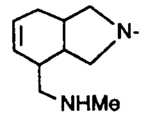 | 168-170 (Zers.) |
| 27 | 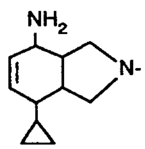 | 231-233 (Zers.) |

Beispiel 28

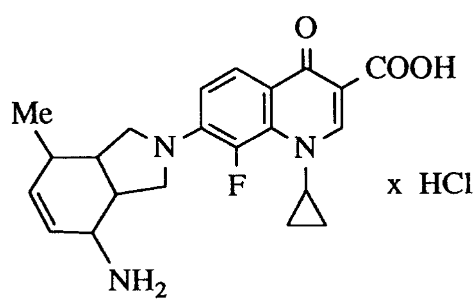

x HCl

7-(4-Amino-7-methyl-1,3,3a,4,7,7a-hexahydroisoindol-2-yl)-1-cyclopropyl-8-fluor-1,4-dihydro-4-oxo-3-chinolincarbonsäurehydrochlorid

Analog zum Beispiel 15 wird bei der Umsetzung von 7-(4-Amino-7-methyl-1,3,3a,4,7,7a-hexahydroisoindol-2-yl)-1-cyclopropyl-8-fluor-1,4-dihydro-4-oxo-3-chinolincarbonsäure die Titelverbindung erhalten. Schmelzpunkt: 262-264 ° C (unter Zersetzung)

Beispiel 29

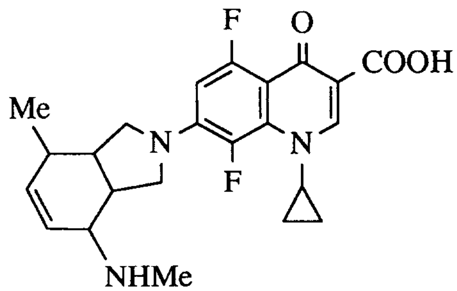

1-Cyclopropyl-5,8-difluor-1,4-dihydro-7-(7-methyl-4-methylamino-1,2,3,3a,4,7,7a-hexahydroisoindol-2-yl)-4-oxo-3-chinolincarbonsäure

1,1 g (4 mmol) 1-Cyclopropyl-5,7,8-trifluor-1,4-dihydro-4-oxo-3-chinolincarbonsäure werden mit 797 mg (4,8 mmol) 7-Methyl-4-methylamino-1,3,3a,4,7,7a-hexahydroisoindol und 896 mg (8 mmol) 1,4-Diazabicyclo-[2.2.2]octan in 40 ml Dimethylsulfoxid zwei Tage bei Raumtemperatur gerührt. Alle flüchtigen Bestandteile werden im Hochvakuum entfernt, der Rückstand mit Acetonitril verrührt und bei ca. 100 °C getrocknet.
Ausbeute: 1,2 g (70 % der Theorie)
Schmelzpunkt: 210-212 °C (unter Zersetzung)

Beispiel 30

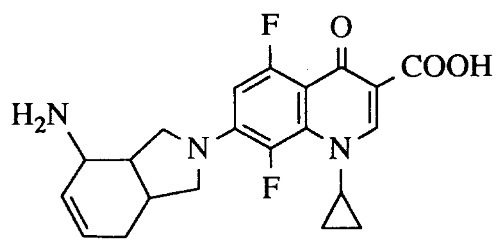

7-(4-Amino-1,3,3a,4,7,7a-hexahydroisoindol-2-yl)-1-cyclopropyl-5,8-difluor-1,4-dihydro-4-oxo-3-chinolincarbonsäure

Analog zum Beispiel 29 wird bei der Umsetzung mit 4-Amino-1,3,3a,4,7,7a-hexahydroisoindol die Titelverbindung erhalten.
Schmelzpunkt: 272-274 °C (unter Zersetzung)

Beispiel 31

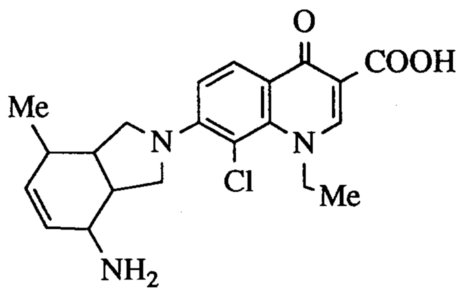

7-(4-Amino-7-methyl-1,3,3a,4,7,7a-hexahydroisoindol-2-yl)-8-chlor-1-ethyl-1,4-dihydro-4-oxo-3-chinolincarbonsäure

270 mg (1 mmol) 8-Chlor-1-ethyl-7-fluor-1,4-dihydro-4-oxo-3-chinolincarbonsäure werden mit 180 mg (1,2 mmol) 4-Amino-7-methyl-1,3,3a,4,7,7a-hexahydroisoindol und 224 mg (2 mmol) 1,4-Diazabicyclo[2.2.2]-octan in 10 ml Dimethylsulfoxid drei Stunden auf 100 ° C erwärmt.

Alle flüchtigen Bestandteile werden im Hochvakuum entfernt, der Rückstand mit Acetonitril verrührt und bei 100 ° C getrocknet.

Ausbeute: 340 mg (85 % der Theorie)

Schmelzpunkt: 164-166 ° C

Beispiel 32

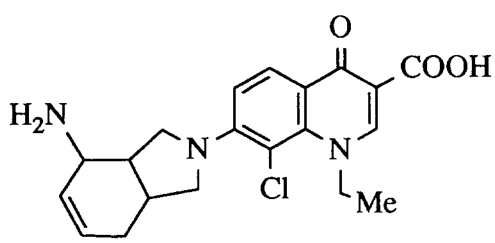

7-(4-Amino-1,3,3a,4,7,7a-hexahydroisoindol-2-yl)-8-chlor-1-ethyl-1,4-dihydro-4-oxo-3-chinolincarbonsäure

Analog zum Beispiel 31 wird bei der Umsetzung mit 4-Amino-1,3,3a,4,7,7a-hexahydroisoindol die Titelverbindung erhalten.

Schmelzpunkt: 194-196 ° C (unter Zersetzung)

Beispiel 33

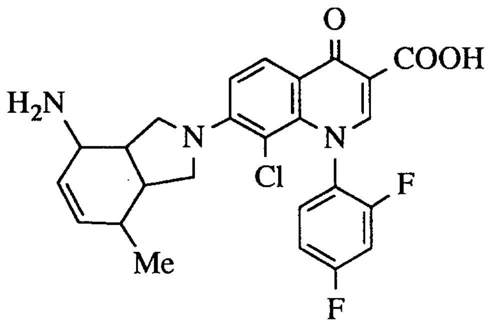

7-(4-Amino-7-methyl-1,3,3a,4,7,7a-hexahydroisoindol-2-yl)-8-chlor-1-(2,4-difluorphenyl)-1,4-dihydro-4-oxo-3-chinolincarbonsäure

Analog zum Beispiel 31 wird bei der Umsetzung von 8-Chlor-7-fluor-1-(2,4-difluorphenyl)-1,4-dihydro-4-oxo-3-chinolincarbonsäure mit 4-Amino-7-methyl-1,3,3a,4,7,7a-hexahydroisoindol die Titelverbindung erhalten.
Schmelzpunkt: 231-233 °C (unter Zersetzung)

Beispiel 34

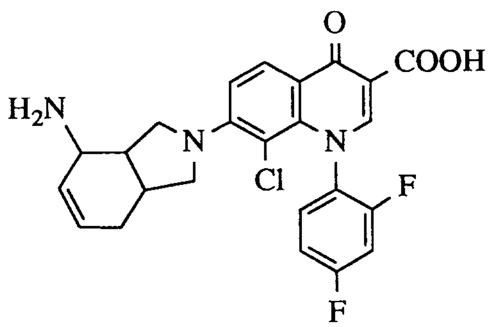

7-(4-Amino-1,3,3a,4,7,7a-hexahydroisoindol-2-yl)-8-chlor-1-(2,4-difluorphenyl)-1,4-dihydro-4-oxo-3-chinolincarbonsäure

Analog zum Beispiel 31 wird bei der Umsetzung von 8-Chlor-7-fluor-1-(2,4-difluorphenyl)-1,4-dihydro-4-oxo-3-chinolincarbonsäure mit 4-Amino-1,3,3a,4,7,7a-hexahydroisoindol die Titelverbindung erhalten.
Schmelzpunkt: 256-258 °C (unter Zersetzung)

Beispiel 35

7-(4-Amino-1,3,3a,4,7,7a-hexahydroisoindol-2-yl)-1-cyclopropyl-1,4-dihydro-4-oxo-1,8-naphthyridin-3-carbonsäurehydrochlorid

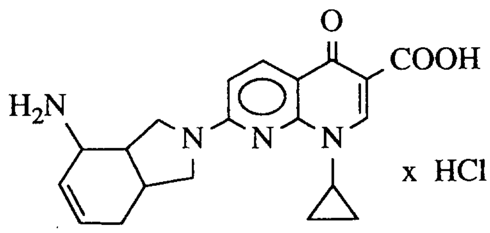

a) 7-(4-Amino-1,3,3a,4,7,7a-hexahydroisoindol-2-yl)-1-cyclopropyl-1,4-dihydro-4-oxo-1,8-naphthyridin-3-carbonsäureethylester

292 mg (1 mmol) 7-Chlor-1-cyclopropyl-1,4-dihydro-4-oxo-1,8-naphthyridin-3-carbonsäureethylester werden mit 0,3 g (2,2 mmol) 4-Amino-1,3,3a,4,7,7a-hexahydroisoindol in einer Mischung aus 3 ml Dimethylformamid und 6 ml Acetonitril zwei Stunden unter Argon bei Raumtemperatur gerührt. Die Lösungsmittel werden im Hochvakuum entfernt und der Rückstand mit Acetonitril verrührt.

Ausbeute: 160 mg (40 % der Theorie)

Schmelzpunkt: 192-193 ° C (unter Zersetzung)

b) 7-(4-Amino-1,3,3a,4,7,7a-hexahydroisoindol-2-yl)-1-cyclopropyl-1,4-dihydro-4-oxo-1,8-naphthyridin-3-carbonsäurehydrochlorid

150 mg (0,38 mmol) des oben erhaltenen Esters werden in 4,2 ml Essigsäure und 3,4 ml 18 %iger Salzsäure zehn Stunden zum Rückfluß erhitzt. Es wird bis zur Trockene einrotiert und bei ca 100 ° C getrocknet.

Ausbeute: 110 mg (73 % der Theorie)

Schmelzpunkt: 268-270 ° C(Zersetzung)

Beispiel 36

7-(4-Amino-7-methyl-1,3,3a,4,7,7a-hexahydroisoindol-2-yl)-1-cyclopropyl-1,4-dihydro-4-oxo-1,8-naphthyridin-3-carbonsäure-hydrochlorid

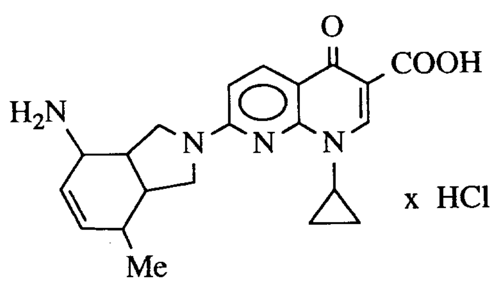

a) 7-(4-Amino-7-methyl-1,3,3a,4,7,7a-hexahydroisoindol-2-yl)-1-cyclopropyl-1,4-dihydro-4-oxo-1,8-naphthyridin-3-carbonsäureethylester

Analog zum Beispiel 35 a. wird bei der Umsetzung mit 4-Amino-7-methyl-1,3,3a,4,7,7a-hexahydroisoindol die Titelverbindung erhalten.

Schmelzpunkt: 172-176 ° C

b) 7-(4-Amino-7-methyl-1,3,3a,4,7,7a-hexahydroisoindol-2-yl)-1-cyclopropyl-1,4-dihydro-4-oxo-1,8-naphthyridin-3-carbonsäurehydrochlorid

Analog zum Beispiel 35 b. wird bei der Umsetzung der oben erhaltenen Verbindung die Titelverbindung erhalten.

Schmelzpunkt: 260-262 ° C

Beispiel 37

7-(4-Amino-1,3,3a,4,7,7a-hexahydroisoindol-2-yl)-2,4-difluorphenyl-1,4-dihydro-4-oxo-1,8-naphthyridin-3-carbonsäurehydrochlorid

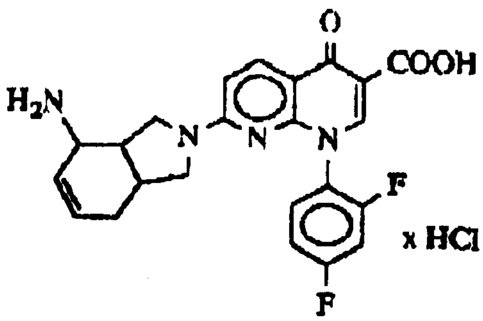

a) 7-(4-Amino-1,3,3a,4,7,7a-hexahydroisoindol-2-yl)-1-(2,4-difluorphenyl)-1,4-dihydro-4-oxo-1,8-naphthyridin-3-carbonsäureethylester
Analog zum Beispiel 35 a. wird bei der Umsetzung von 7-Chlor-1-(2,4-difluorphenyl)-1,4-dihydro-4-oxo-1,8-naphthyridin-3-carbonsäureethylester mit 4-Amino-1,3,3a,4,7,7a-hexahydroisoindol die Titelverbindung erhalten.
Schmelzpunkt: 178-180 ° C
b) 7-(4-Amino-1,3,3a,4,7,7a-hexahydroisoindol-2-yl)-1-(2,4-diflourphenyl)-1,4-dihydro-4-oxo-1,8-naphthyridin-3-carbonsäurehydrochlorid
Analog zum Beispiel 35 b. wird bei der Umsetzung der oben erhaltenen Verbindung die Titelverbindung erhalten.
Schmelzpunkt: 254-256 ° C

Beispiel 38

7-(4-Amino-7-methyl-1,3,3a,4,7,7a-hexahydroisoindol-2-yl)-1-(2,4-difluorphenyl)-1,4-dihydro-4-oxo-1,8-naphthyridin-3-carbonsäurehydrochlorid

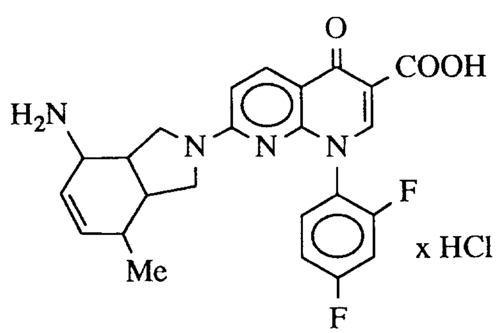

a) 7-(4-Amino-7-methyl-1,3,3a,4,7,7a-hexahydroisoindol-2-yl)-1-(2,4-difluorphenyl)1,4-dihydro-4-oxo-1,8-naphthyridin-3-carbonsäureethylester
Analog zum Beispiel 35 a. wird bei der Umsetzung von 7-Chlor-1-(2,4-difluorphenyl)-1,4-dihydro-4-oxo-1,8-naphthyridin-3-carbonsäureethylester mit 4-Amino-7-methyl-1,3,3a,4,7,7a-hexahydroisoindol die Titelverbindung erhalten.
Schmelzpunkt: 143-144 ° C

b) 7-(4-Amino-7-methyl-1,3,3a,4,7,7a-hexahydroisoindol-2-yl)-1-(2,4-difluorphenyl)-1,4-dihydro-4-oxo-1,8-naphthyridin-3-carbonsäurehydrochlorid

Analog zum Beispiel 35 b. wird bei der Umsetzung der oben erhaltenen Verbindung die Titelverbindung erhalten.

Schmelzpunkt: 244-245 °C

Beispiel 39

7-(4-Amino-1,3,3a,4,7,7a-hexahydroisoindol-2-yl)-8-chlor-1-(cis-2-fluorcyclopropyl)-1,4-dihydro-4-oxo-3-chinolincarbonsäure

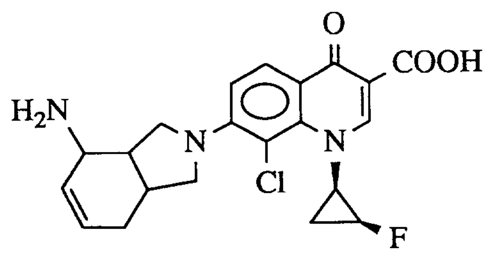

299 mg (1 mmol) 8-Chlor-7-fluor-1-(cis-2-fluorcyclopropyl)-1,4-dihydro-4-oxo-3-chinolincarbonsäure werden mit 165 mg (1,2 mmol) 4-Amino-1,3,3a,4,7,7a-hexahydroisoindol und 224 mg (2 mmol) 1,4-Diazabicyclo[2.2.2]octan in 10 ml Dimethylsulfoxid über Nacht bei Raumtemperatur gerührt. Alle flüchtigen Bestandteile werden im Hochvakuum entfernt, der Rückstand mit Acetonitril verrührt und bei 100 °C getrocknet.

Ausbeute: 400 mg (95 % der Theorie)

Schmelzpunkt: 159-161 °C

Beispiel 40

7-(4-Amino-7-methyl-1,3,3a,4,7,7a-hexahydroisoindol-2-yl)-8-chlor-1-(cis-2-fluorcyclopropyl)-1,4-dihydro-4-oxo-3-chinolincarbonsäure

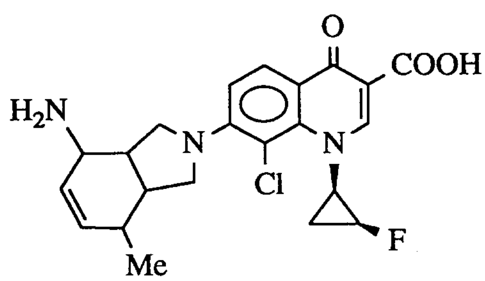

Analog zum Beispiel 39 wird bei der Umsetzung von 8-Chlor-7-fluor-1-(cis-2-fluorcyclopropyl)-1,4-dihydro-4-oxo-3-chinolincarbonsäure mit 4-Amino-7-methyl-1,3,3a,4,7,7a-hexahydroisoindol die Titelverbindung erhalten.

Schmelzpunkt: 168-170 °C

Beispiel 41

7-(4-Amino-1,3,3a,4,7,7a-hexahydroisoindol-2-yl)-8-chlor-1-(trans-2-fluorcyclopropyl)-1,4-dihydro-4-oxo-3-chinolincarbonsäure

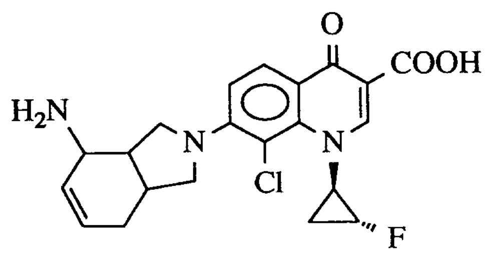

299 mg (1 mmol) 8-Chlor-7-fluor-1-(trans-2-fluorcyclopropyl)-1,4-dihydro-4-oxo-3-chinolincarbonsäure werden mit 165 mg (1,2 mmol) 4-Amino-1,3,3a,4,7,7a-hexahydroisoindol und 224 mg (2 mmol) 1,4-Diazabicyclo[2.2.2]octan in 10 ml Dimethylsulfoxid über Nacht bei Raumtemperatur gerührt. Alle flüchtigen Bestandteile werden im Hochvakuum entfernt, der Rückstand mit Acetonitril verrührt und bei 100 °C getrocknet.
Ausbeute: 400 mg (95 % der Theorie)
Schmelzpunkt: 181-183 °C

Beispiel 42

7-(4-Amino-7-methyl-1,3,3a,4,7,7a-hexahydroisoindol-2-yl)-8-chlor-1-(trans-2-fluor-cyclopropyl)-1,4-dihydro-4-oxo-3-chinolincarbonsäure

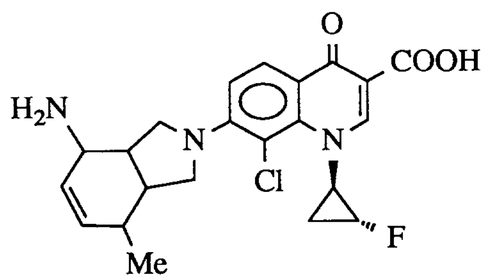

Analog zum Beispiel 41 wird bei der Umsetzung von 8-Chlor-7-fluor-1-(trans-2-fluorcyclopropyl)-1,4-dihydro-4-oxo-3-chinolincarbonsäure mit 4-Amino-7-methyl-1,3,3a,4,7,7a-hexahydroisoindol die Titelverbindung erhalten.
Schmelzpunkt: 177-179 °C

Beispiel 43

7-(4-Amino-1,3,3a,4,7,7a-hexahydroisoindol-2-yl)-8-fluor-1-(cis-2-fluorcyclopropyl)-1,4-dihydro-4-oxo-3-chinolincarbonsäure

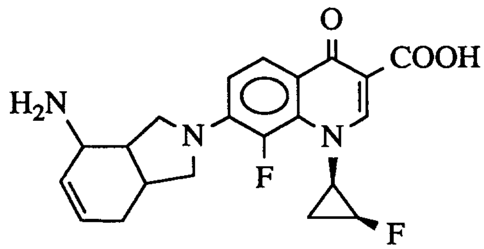

283 mg (1 mmol) 7,8-Difluor-1-(cis-2-fluorcyclopropyl)-1,4-dihydro-4-oxo-3-chinolincarbonsäure werden mit 165 mg (1,2 mmol) 4-Amino-1,3,3a,4,7,7a-hexahydroisoindol und 224 mg (2 mmol) 1,4-Diazabicyclo[2.2.2]-octan in 10 ml Dimethylsulfoxid über Nacht bei Raumtemperatur gerührt. Alle flüchtigen Bestandteile werden im Hochvakuum entfernt, der Rückstand mit Ethanol verrührt und bei 100 °C getrocknet.
Ausbeute: 330 mg (82 % der Theorie)
Schmelzpunkt: 244-246 °C (unter Zersetzung)

Beispiel 44

7-(4-Amino-7-methyl-1,3,3a,4,7,7a-hexahydroisoindol-2-yl)-8-fluor-1-(cis-2-fluorcyclopropyl)-1,4-dihydro-4-oxo-3-chinolincarbonsäure

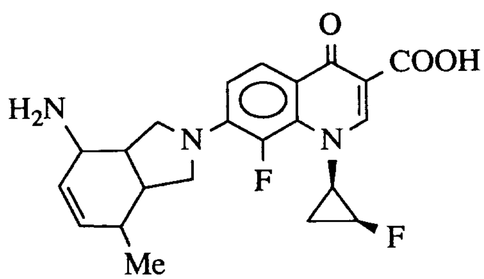

Analog zum Beispiel 43 wird bei der Umsetzung von 7,8-Difluor-1-(cis-2-fluorcyclopropyl)-1,4-dihydro-4-oxo-3-chinolincarbonsäure mit 4-Amino-7-methyl-1,3,3a,4,7,7a-hexahydroisoindol die Titelverbindung erhalten.
Schmelzpunkt: 204-206 °C (unter Zersetzung)

Beispiel 45

7-(4-Amino-1,3,3a,4,7,7a-hexahydroisoindol-2-yl)-1-cyclopropyl-8-fluor-5-trifluormethyl-1,4-dihydro-4-oxo-3-chinolincarbonsäure

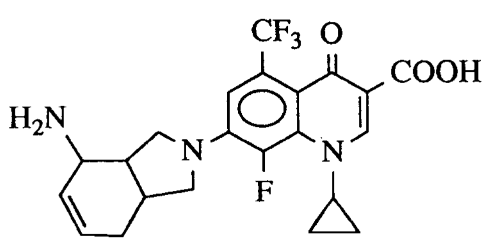

333 mg (1 mmol) 1-Cyclopropyl-7,8-difluor-5-trifluormethyl-1,4-dihydro-4-oxo-3-chinolincarbonsäure werden mit 165 mg (1,2 mmol) 4-Amino-1,3,3a,4,7,7a-hexahydroisoindol und 224 mg (2 mmol) 1,4-Diazabicyclo-[2.2.2]octan in 10 ml Dimethylsulfoxid über Nacht bei Raumtemperatur gerührt. Alle flüchtigen Bestandteile werden im Hochvakuum entfernt, der Rückstand mit Acetonitril verrührt und bei 100 ° C getrocknet.
Ausbeute: 330 mg (73 % der Theorie)
Schmelzpunkt: 248-249 ° C (unter Zersetzung)

Beispiel 46

7-(4-Amino-7-methyl-1,3,3a,4,7,7a-hexahydroisoindol-2-yl)-1-cyclopropyl-8-fluor-5-trifluormethyl-1,4-dihydro-4-oxo-3-chinolincarbonsäure

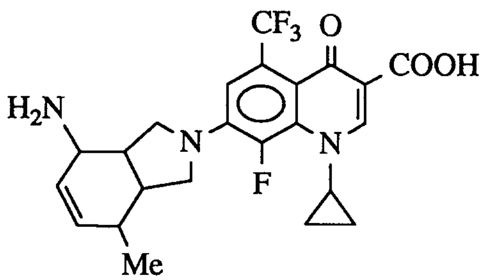

Analog zum Beispiel 45 wird bei der Umsetzung von 1-Cyclopropyl-7,8-difluor-5-trifluormethyl-1,4-dihydro-4-oxo-3-chinolincarbonsäure mit 4-Amino-7-methyl-1,3,3a,4,7,7a-hexahydroisoindol die Titelverbindung erhalten.
Schmelzpunkt: 242-244 ° C (unter Zersetzung)

Beispiel 47

7-(4-Amino-1,3,3a,4,7,7a-hexahydroisoindol-2-yl)-1-cyclopropyl-5-fluor-8-trifluormethyl-1,4-dihydro-4-oxo-3-chinolincarbonsäure

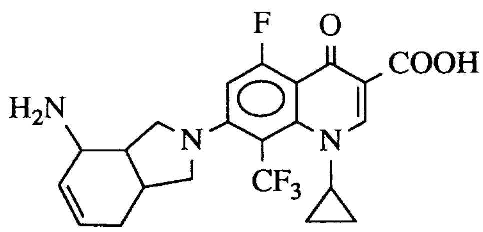

Analog zum Beispiel 45 wird bei der Umsetzung von 1-Cyclopropyl-5,7-difluor-8-trifluormethyl-1,4-dihydro-4-oxo-3-chinolincarbonsäure mit 4-Amino-1,3,3a,4,7,7a-hexahydroisoindol die Titelverbindung erhalten.
Schmelzpunkt: 238-240 ° C (unter Zersetzung)

Beispiel 48

7-(4-Amino-7-methyl-1,3,3a,4,7,7a-hexahydroisoindol-2-yl)-1-cyclopropyl-5-fluor-8-trifluormethyl-1,4-dihydro-4-oxo-3-chinolincarbonsäure

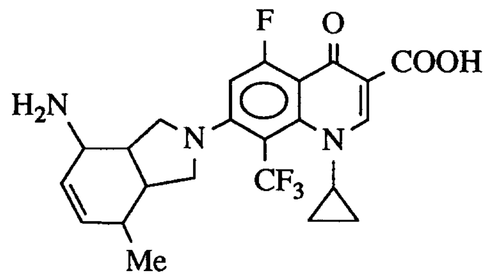

Analog zum Beispiel 45 wird bei der Umsetzung von 1-Cyclopropyl-5,7-difluor-8-trifluormethyl-1,4-dihydro-4-oxo-3-chinolincarbonsäure mit 4-Amino-7-methyl-1,3,3a,4,7,7a-hexahydroisoindol die Titelverbindung erhalten.
Schmelzpunkt: 234-236 ° C (unter Zersetzung)

Beispiel 49

7-(4-Amino-7-methyl-1,3,3a,4,7,7a-hexahydroisoindol-2-yl)-1-cyclopropyl-8-trifluormethyl-1,4-dihydro-4-oxo-3-chinolincarbonsäure

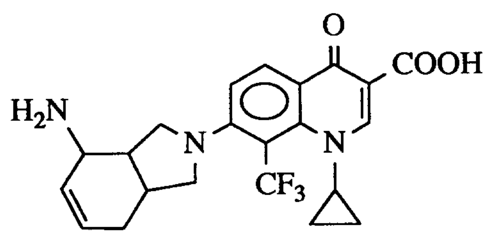

315 mg (1 mmol) 1-Cyclopropyl-7-fluor-8-trifluormethyl-1,4-dihydro-4-oxo-3-chinolincarbonsäure werden mit 165 mg (1,2 mmol) 4-Amino-1,3,3a,4,7,7a-hexahydroisoindol und 224 mg (2 mmol) 1,4-Diazabicyclo[2.2.2]-octan in 10 ml Dimethylsulfoxid zwei Stunden bei 100 ° C gerührt Alle flüchtigen Bestandteile werden im Hochvakuum entfernt, der Rückstand mit Acetonitril verrührt und bei 100 ° C getrocknet.
Ausbeute: 280 mg (65 % der Theorie)
Schmelzpunkt: 168-170 ° C (unter Zersetzung)

Beispiel 50

1-Cyclopropyl-5,8-difluor-1,4-dihydro-7-(4-methylamino-1,2,3,3a,4,7,7a-hexahydroisoindol-2-yl)-4-oxo-3-chinolincarbonsäure

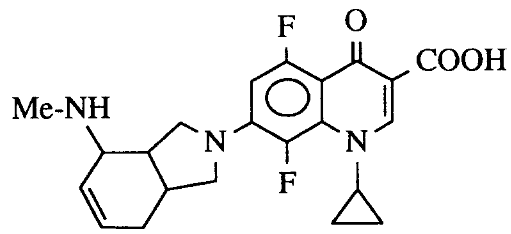

4,245 g (0,015 mol) 1-Cyclopropyl-5,7,8-trifluor-1,4-dihydro-4-oxo-3-chinolincarbonsäure werden mit 2,736 g (0,018 mol) 3-Methylamino-1,3,3a,4,7,7a-hexahydroisoindol und 3,36 g (0,03 mol) 1,4-Diazabicyclo[2.2.2]-octan in 150 ml Dimethylsulfoxid über Nacht bei Raumtemperatur gerührt. Alle flüchtigen Komponenten werden im Hochvakuum entfernt und der Rückstand mit Acetonitril verrührt.
Ausbeute: 5,5 g (88 % der Theorie)
Schmelzpunkt: 238-240 °C (unter Zersetzung)

Beispiel 51

5-Amino-1-cyclopropyl-8-fluor-1,4-dihydro-7-(4-methylamino-1,3,3a,4,7,7a-hexahydroisoindol-2-yl)-4-oxo-3-chinolincarbonsäure

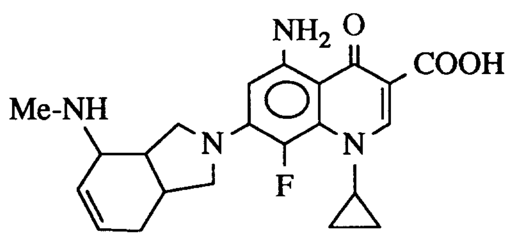

2 g (4,8 mmol) 1-Cyclopropyl-5,8-difluor-1,4-dihydro-7-(4-methylamino-1,3,3a,4,7,7a-hexahydroisoindol-2yl)-4-oxo-3-chinolincarbonsäure werden in 200 ml Dimethylsulfoxid in einem Autoklaven vorgelegt. Nach Zugabe von 5 ml flüssigen Ammoniak wird 9 Stunden bei 140 °C und 6 bar gerührt. Alle flüchtigen Komponenten werden im Vakuum entfernt und der Rückstand mit Ethanol verrührt.
Ausbeute: 0,7 g (35,5 % der Theorie)
Schmelzpunkt: 156-158 °C (unter Zersetzung)

**Patentansprüche**

1. Chinolon- und Naphthyridoncarbonsäurederivate der allgemeinen Formel (I)

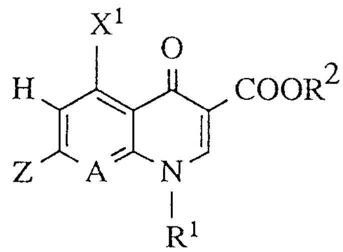 (I),

in welcher

- $R^1$ für gegebenenfalls durch Hydroxy, Halogen oder $C_1$-$C_3$-Alkoxy substituiertes geradkettiges oder verzweigtes $C_1$-$C_4$-Alkyl, für gegebenenfalls durch $C_1$-$C_3$-Alkyl oder Halogen substituiertes $C_3$-$C_6$-Cycloalkyl, $C_2$-$C_4$-Alkenyl, ferner für $C_1$-$C_3$-Alkoxy, Amino, Monoalkylamino mit 1 bis 3 C-Atomen, Dialkylamino mit 1 bis 3 C-Atomen je Alkylgruppe oder für gegebenenfalls durch Halogen ein- bis dreifach substituiertes Phenyl steht,
- $R^2$ für Wasserstoff, gegebenenfalls durch Hydroxy, Methoxy, Amino, Methylamino oder Dimethylamino substituiertes Alkyl mit 1 bis 4 Kohlenstoffatomen oder (5-Methyl-2-oxo-1,3-dioxol-4-yl)-methyl,
- $X^1$ für Wasserstoff, Halogen, Amino, Methyl oder Trifluormethyl,
- Z für Reste der Strukturen

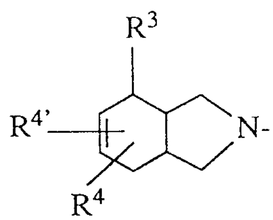 , 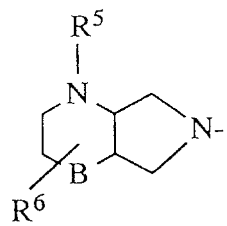

steht,
worin

- $R^3$ für Wasserstoff, Hydroxy, $-NR^7R^8$, Hydroxymethyl oder $-CH_2-NR^7R^8$ steht,
  wobei
- $R^7$ Wasserstoff, gegebenenfalls durch Hydroxy substituiertes $C_1$-$C_3$-Alkyl, Alkoxycarbonyl mit 1 bis 4 C-Atomen im Alkoxyteil oder $C_1$-$C_3$-Acyl und
- $R^8$ Wasserstoff oder Methyl bedeutet,
- $R^4$ für Wasserstoff, geradkettiges oder verzweigtes $C_1$-$C_3$-Alkyl oder Cyclopropyl,
- $R^{4'}$ für Wasserstoff oder Methyl,
- $R^5$ für Wasserstoff oder Methyl,
- $R^6$ für Wasserstoff, Methyl oder Reste der Strukturen $-CH=CH-CO_2R^{5'}$,$-CH_2-CH_2-CO_2R^{5'}$, $-CH_2-CO-CH_3$, $-CH_2-CH_2-CN$,
- $R^{5'}$ für Methyl oder Ethyl,
- B für $-CH_2-$, O oder eine direkte Bindung steht und
- A für N oder C-$R^9$ steht,
  worin
- $R^9$ für H, Halogen, Methyl, Trifluormethyl, Vinyl, Ethinyl, Hydroxy oder Methoxy steht oder auch gemeinsam mit $R^1$ eine Brücke der Struktur

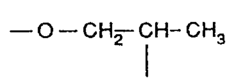 , 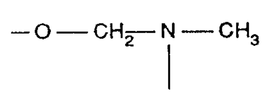 , 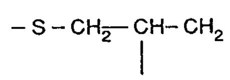 oder 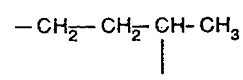

bilden kann,

in Form von Racematen oder als enantiomerenreine Verbindungen, deren pharmazeutisch verwendbaren Hydrate und Säureadditionsalze sowie die Alkali-, Erdalkali-, Silber- und Guanidiniumsalze der

zugrundeliegenden Carbonsäuren.

2. Chinolon- und Naphthyridoncarbonsäurederivate gemäß Anspruch 1, dadurch gekennzeichnet, daß in Formel (I)
   - $R^1$ für gegebenenfalls durch Hydroxy oder Fluor substituiertes $C_1$-$C_2$-Alkyl, für gegebenenfalls durch Fluor substituiertes $C_3$-$C_5$-Cycloalkyl, Vinyl, Amino, Monoalkylamino mit 1 bis 2 C-Atomen, Dialkylamino mit 1 bis 2 C-Atomen je Alkylgruppe oder für gegebenenfalls durch Halogen ein- bis zweifach substituiertes Phenyl steht,
   - $R^2$ für Wasserstoff, gegebenenfalls durch Amino oder Dimethylamino substituiertes Alkyl mit 1 bis 2 Kohlenstoffatomen oder (5-Methyl-2-oxo-1,3-dioxol-4-yl)-methyl,
   - $X^1$ für Wasseistoff, Fluor, Chlor, Trifluormethyl oder Amino,
   - Z für Reste der Strukturen

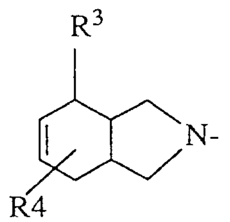 , 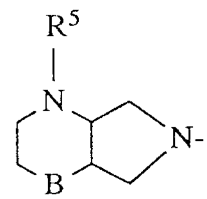

steht,
worin
   - $R^3$ für Wasserstoff, Hydroxy, $-NR^7R^8$, Hydroxymethyl oder $-CH_2-NR^7R^8$ steht,
     wobei
   - $R^7$ Wasserstoff, gegebenenfalls durch Hydroxy substituiertes $C_1$-$C_2$-Alkyl, Alkoxycarbonyl mit 1 bis 4 C-Atomen im Alkoxyteil oder $C_1$-$C_3$-Acyl und
   - $R^8$ Wasserstoff oder Methyl bedeutet,
   - $R^4$ für Wasserstoff, geradkettiges oder verzweigtes $C_1$-$C_3$-Alkyl oder Cyclopropyl,
   - $R^5$ für Wasserstoff oder Methyl,
   - B für $-CH_2-$, O oder eine direkte Bindung steht und
   - A für N oder C-$R^9$ steht,
     worin
   - $R^9$ für H, Chlor, Fluor, Methyl, Trifluormethyl, Hydroxy oder Methoxy steht oder auch gemeinsam mit $R^1$ eine Brücke der Struktur

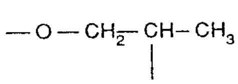 oder 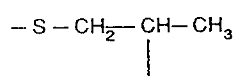

bilden kann.

3. Chinolon- und Naphthyridoncarbonsäurederivate gemäß Anspruch 1, dadurch gekennzeichnet, daß in Formel (I)
   - $R^1$ für Methyl, Ethyl, gegebenenfalls durch Fluor substituiertes Cyclopropyl oder für gegebenenfalls durch Fluor ein- oder zweifach substituiertes Phenyl steht,
   - $R^2$ für Wasserstoff, Methyl oder Ethyl,
   - $X^1$ für Wasserstoff, Fluor, Trifluormethyl oder Amino,
   - Z für Reste der Strukturen

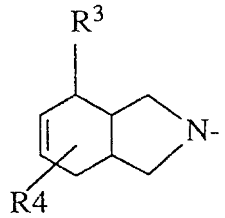

,

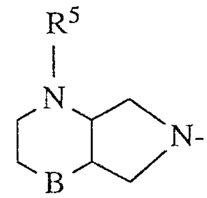

steht,

worin

- $R^3$ für Wasserstoff, Hydroxy, $-NR^7R^8$, Hydroxymethyl oder $-CH_2-NR^7R^8$ steht,
  wobei
- $R^7$ Wasserstoff, Methyl, Alkoxycarbonyl mit 1 bis 4 C-Atomen im Alkoxyteil oder $C_1$-$C_3$-Acyl und
- $R^8$ Wasserstoff oder Methyl bedeutet,
- $R^4$ für Wasserstoff, geradkettiges oder verzweigtes $C_1$-$C_3$-Alkyl oder Cyclopropyl,
- $R^5$ für Wasserstoff oder Methyl,
- B für $-CH_2-$, O oder eine direkte Bindung steht und
- A für N oder C-$R^9$ steht,
  worin
- $R^9$ für H, Chlor, Fluor, Methyl, Methoxy, Trifluormethyl steht oder auch gemeinsam mit $R^1$ eine Brücke der Struktur

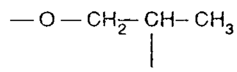

bilden kann.

**4.** Arzneimittel enthaltend Chinolon- und Naphthyridoncarbonsäurederivate gemäß, Ansprüchen 1 bis 3.

**5.** Antibakterielle Mittel enthaltend Chinolon- und Naphthyridoncarbonsäurederivate gemäß Ansprüchen 1 bis 3.

**6.** Verwendung von Chinolon- und Naphthyridoncarbonsäurederivate gemäß Ansprüchen 1 bis 3 bei der Herstellung von Arzneimitteln.

**7.** Verwendung von Chinolon- und Naphthyridoncarbonsäurederivate gemäß Ansprüchen 1 bis 3 zum Materialschutz.

**8.** Futtermittel und Prämixe enthaltend Chinolon- und Naphthyridoncarbonsäurederivate gemäß Ansprüchen 1 bis 3.

**9.** Verfahren zur Herstellung von Chinolon- und Naphthyridoncarbonsäurederivate gemäß Ansprüchen 1 bis 3, dadurch gekennzeichnet, daß Verbindungen der Formel (II)

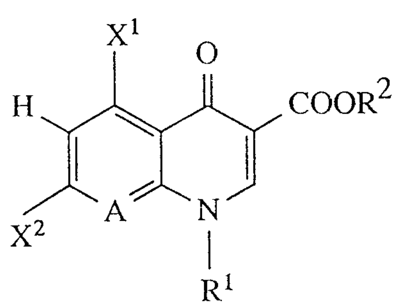

(II),

in welcher

$R^1$, $R^2$, $X^1$ und A die Bedeutung gemäß Ansprüchen 1 bis 3 haben und
$X^2$ für Halogen steht,

mit Verbindungen der Formel (III)

Z-H (III),

in welcher

Z die Bedeutung gemäß Ansprüchen 1 bis 3 hat,

gegebenenfalls in Gegenwart von Säurefängern umgesetzt werden.

**10.** Verbindungen aus der Gruppe bestehend aus
1-Cyclopropyl-7-fluor-1,4-dihydro-4-oxo-3-chinolincarbonsäure,
8-Chlor-1-cyclopropyl-7-fluor-1,4-dihydro-4-oxo-3-chinolincarbonsäure,
1-Cyclopropyl-5,7,8-trifluor-1,4-dihydro-4-oxo-3-chinolincarbonsäure,
1-Ethyl-7,8-difluor-1,4-dihydro-4-oxo-3-chinolincarbonsäure,
7,8-Difluor-1-(2,4-difluorphenyl)-1,4-dihydro-4-oxo-3-chinolincarbonsäure,
8-Chlor-1-ethyl-7-fluor-1,4-dihydro-4-oxo-3-chinolincarbonsäure,
8-Chlor-7-fluor-1-(2,4-difluorphenyl)-1,4-dihydro-4-oxo-3-chinolincarbonsäure,
1-Cyclopropyl-7-fluor-1,4-dihydro-8-methyl-4-oxo-3-chinolincarbonsäure,
7-Chlor-1-cyclopropyl-1,4-dihydro-4-oxo-1,8-naphthyridin-3-carbonsäureethylester,
7-Chlor-1-(2,4-difluorphenyl)-1,4-dihydro-4-oxo-1,8-naphthyridin-3-carbonsäureethylester,
8-Chlor-7-fluor-1-(trans-2-fluorcyclopropyl)-1,4-dihydro-4-oxo-3-chinolincarbonsäure,
7,8-Difluor-1-(cis-2-fluorcyclopropyl)-1,4-dihydro-4-oxo-3-chinolincarbonsäure,
1-Cyclopropyl-7-fluor-8-trifluormethyl-1,4-dihydro-4-oxo-3-chinolincarbonsäure,
1-Cyclopropyl-5,7-difluor-8-trifluormethyl-1,4-dihydro-4-oxo-3-chinolincarbonsäure,
1-Cyclopropyl-7,8-difluor-5-trifluormethyl-1,4-dihydro-4-oxo-3-chinolincarbonsäure.

Europäisches Patentamt

# EUROPÄISCHER RECHERCHENBERICHT

Nummer der Anmeldung
EP 94 10 0204

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.5) |
|---|---|---|---|
| A | EP-A-0 520 240 (BAYER AG)<br>* Ansprüche * | 1,4,5 | C07D471/04<br>A61K31/47<br>C07D401/04<br>C07D487/04<br>C07D498/04 |
| A | EP-A-0 516 861 (BANYU PHARMACEUTICAL CO.)<br>* Seite 3; Anspruche * | 1,4,5 | |
| D,A | EP-A-0 393 400 (WAKUNAGA SEIYAKU KABUSHIKI KAISHA)<br>* Ansprüche * | 1,4,5 | |
| A | EP-A-0 429 304 (SHIONOGI SEIYAKU KABUSHIKI KAISHA)<br>* Ansprüche * | 1,4,5 | |
| D,A | US-A-4 416 884 (OTSUKA PHARMACEUTICAL CO.)<br>* Ansprüche * | 1,4,5 | |
| P,A | EP-A-0 550 903 (BAYER AG)<br>* Ansprüche * | 1,4,5 | |

RECHERCHIERTE SACHGEBIETE (Int.Cl.5)

C07D
A61K

Der vorliegende Recherchenbericht wurde für alle Patentansprüche erstellt

| Recherchenort | Abschlußdatum der Recherche | Prüfer |
|---|---|---|
| DEN HAAG | 5. April 1994 | Van Bijlen, H |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P04C03)